(19) Europäisches Patentamt / European Patent Office / Office européen des brevets

(11) EP 3 295 820 B1

# (12) EUROPEAN PATENT SPECIFICATION

(45) Date of publication and mention of the grant of the patent: **22.05.2019 Bulletin 2019/21**

(51) Int Cl.: ***A44C 15/00*** $^{(2006.01)}$ ***F21V 8/00*** $^{(2006.01)}$ *A45D 8/00* $^{(2006.01)}$

(21) Application number: **16188964.7**

(22) Date of filing: **15.09.2016**

## (54) COLORED HAIR ORNAMENT, METHOD AND KIT THEREOF

GEFÄRBTER HAARSCHMUCK, VERFAHREN UND KIT DAFÜR

PARURE DE CHEVEUX COLORÉS, PROCÉDÉ ET SON KIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.03.2018 Bulletin 2018/12**

(73) Proprietor: **Noxell Corporation**
**Hunt Valley, MD 21030-2098 (US)**

(72) Inventors:
- **HERRLEIN, Mathias**
  **65824 Schwalbach am Taunus (DE)**
- **FORGIONE, Marianna**
  **65824 Schwalbach am Taunus (DE)**
- **SCHAEFER, Tatjana**
  **65824 Schwalbach am Taunus (DE)**
- **AMANN, Mathias**
  **61476 Kronberg/Taunus (DE)**

(74) Representative: **Bandpay & Greuter**
**30, rue Notre-Dame des Victoires**
**75002 Paris (FR)**

(56) References cited:
**EP-A1- 3 058 934**
**WO-A2-2006/092598**
**US-A1- 2003 156 429**
**US-A1- 2005 068 791**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to the field of colored hair ornament, which comprises a light source, a plurality of light-transmissive filaments, wherein one or more light-transmissive filaments of the plurality of light transmissive filaments are coated with a hair coloring composition. It also relates to a method for customizing the colored hair ornament and a kit.

### BACKGROUND OF THE INVENTION

Decorative hair ornaments are well-known and include polished stones, feathers, thread, fabric, metal and plastic. Only recently have hair ornaments began incorporating electrical components, such as fiber optics, and light sources to improve the aesthetic appeal of such hair ornaments, see for instance US Patent US 3,675,005, or US 2003/156429 A1. Hair ornaments are mostly used to provide a relatively sophisticated or fancy hairstyle.

Traditional hair ornaments utilizing fiber optics and light sources are characterized by a point of light, or points of light, at the end(s) of the fiber optic(s). A light source near a bundle of fiber optics shines a light through the length of each fiber optic until it ultimately exits from a distal end of the fiber optic. A major objective of these traditional hair ornaments is to prevent the light from exiting the fiber optic (also known as "bleed out"), except from the end of the fiber optic. Bleed-out light is light that is traveling in the cladding or slightly imperfectly down the core of the fiber. At some point this light will leave the fiber, since it is not in one of the modes of the wave guide.

Such hair ornaments provide a luminous hairstyle but often the brilliant aspect of the hair ornament does not match with the hair color of the consumer. Hence, there is a need to improve the existing hair ornament to provide additional hair styling benefits to the consumer.

### SUMMARY OF THE INVENTION

A colored hair ornament is provided and comprises a light source; and a plurality of light-transmissive filaments. Each light-transmissive filament has a proximal end, a distal end and a side wall. The proximal end of each light-transmissive filament is arranged to receive light from the light source. One or more light-transmissive filaments of the plurality of light-transmissive filaments are coated with a hair coloring composition. The hair coloring composition comprises a film-forming aminosilicone polymer and a hair coloring agent.

A method of customizing a colored hair ornament is provided and comprises at least one or any combination of two or more of:

mechanically altering the surface of at least one of the plurality of light-transmissive filaments;
cutting at least one of the plurality of light-transmissive filaments;
applying a hair coloring agent or a hair coloring composition to the surface of at least one of the plurality of light-transmissive filaments; and
applying a heat treatment to at least one of the plurality of light-transmissive filaments,
wherein said customizing is preferably performed after attaching the hair ornament to the head of the wearer.

A kit is provided and comprises:

(a) a hair ornament wherein the hair ornament comprises a light source and a plurality of light-transmissive filaments; wherein each light-transmissive filament has a proximal end, a distal end and a side wall, wherein the proximal end of each light-transmissive filament is arranged to receive light from the light source; and
(b) a hair coloring composition, wherein the hair coloring composition comprises a film-forming aminosilicone polymer and a hair coloring agent;
(c) optionally an applicator to coat the hair coloring composition to one or more light-transmissive filaments of the plurality of light-transmissive filaments.

### BRIEF DESCRIPTION OF THE DRAWINGS

While the specification concludes with claims particularly pointing out and distinctly claiming the present invention, it is believed that the same will be better understood from the following description read in conjunction with the accompanying drawings in which:

Fig. 1 is a schematic drawing of a light-transmissive filament according to an embodiment of the invention;
Fig. 2 is a schematic drawing of a light-transmissive filament according to an embodiment of the invention;
Fig. 3 is a schematic drawing of a light-transmissive filament according to an embodiment of the invention;
Fig. 4 is a schematic drawing of a light-transmissive filament according to an embodiment of the invention;
Fig. 5 is a schematic block diagram of a colored hair ornament according to an embodiment;

It should be noted that these figures are diagrammatic and not drawn to scale. Relative dimensions and proportions of parts of these figures have been shown exaggerated or reduced in size, for the sake of clarity and convenience in the drawings.

DETAILED DESCRIPTION OF THE INVENTION

Definitions and general

In this document, including in all embodiments of all aspects of the present invention, the following definitions apply unless specifically stated otherwise.

All percentages are by weight (w/w) of the total composition, unless otherwise specified. All ratios are weight ratios, unless otherwise specified. "wt%" means percentage by weight. References to 'parts' e.g. a mixture of 1 part X and 3 parts Y, is a ratio by weight.

"QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about".

All measurements are understood to be made at 20°C and at ambient conditions, where "ambient conditions" means at 1 atmosphere (atm) of pressure and at 65% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number means "gram" or "grams". All amounts as they pertain to listed ingredients are based on the active level ('solids') and do not include carriers or by-products that may be included in commercially available materials.

Herein, "comprising" means that other steps and other ingredients can be in addition. "Comprising" encompasses the terms "consisting of' and "consisting essentially of'. The hair coloring compositions, methods, uses, and kits of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition. For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "substantially free of' as used herein means less than 1%, less than 0.8%, less than 0.5%, less than 0.3%, or less than an immaterial amount of by total weight of the composition.

"Viscosity" is measured at 23°C using a HAAKE Rotation Viscometer VT 550 with cooling/heating vessel and sensor systems according to DIN 53019 at a shear rate of 12.9 $s^{-1}$.

The term "molecular weight" or "M.Wt." as used herein refers to the weight average molecular weight unless otherwise stated. The weight average molecular weight may be measured by gel permeation chromatography.

The term "Polymer" as used herein means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e.

on a living body or may be "non-living" i.e. in a wig, hairpiece or other aggregation of non-living keratinous fibers. Mammalian, preferably human hair is preferred.

By "hair coloring composition", it is meant a composition suitable for changing the color of hair and/or the light-transmissive filament. The hair coloring composition is referred hereinafter as "the composition", unless otherwise specified. The hair coloring composition can comprise a film-forming aminosilicone polymer and a hair coloring agent. The term "film-forming aminosilicone polymer" as used herein means an aminosilicone polymer which is in a position to deposit a polymer film on the hair. The term "cosmetically acceptable" as used herein means that the compositions, or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

The term "separately packaged" as used herein means any form of packaging that prevents a first composition from coming into physical contact, or admixing, with a second composition. "Separately packaged" may mean that the individual first, and second compositions are packaged in separate containers, or alternatively in a single container partitioned such that the first and second compositions are not in physical contact.

The term "kit" as used herein means a packaging unit comprising a plurality of components i.e. a kit of parts. An example of a kit is, for example, a first composition, and a separately packaged second composition. Another kit may comprise application instructions comprising a method and a first and second compositions.

A colored hair ornament is provided and comprises a light source, a plurality of light-transmissive filaments. The one or more light-transmissive filaments of the plurality of light-transmissive filaments are coated with a hair coloring composition. The hair coloring composition comprises a film-forming aminosilicone polymer and a hair coloring agent.

The present inventors have recognized that it would be desirable to provide a colored hair ornament having a light-transmissive filament which is versatile enough to create a line of light, or a point of light, or a light of light optionally in addition to a point of light. The different aspects of the light-transmissive filament can provide an elaborated and attractive hair styling for sophisticated hairdresses.

At the same time, the present inventors have recognized that when the one or more light-transmissive filaments of the plurality of light-transmissive filaments are coated with a hair coloring composition, the brilliant aspect of the colored hair ornament needs to match with the hair color of the consumer. It can be the natural hair color or the color of the dyed hair fibers of the consumer. The one or more light-transmissive filaments of the plurality of light-transmissive filaments as set out hereinafter are not dyed or painted with a flexible paint on their outer surfaces in order to camouflage the one or more light-transmissive filaments while being worn in the hair, or to prevent the bleed-out of the transmitted light. The one or more light-transmissive filaments of the plurality of light-transmissive filaments are coated with a hair coloring composition which comprises a film-forming aminosilicone polymer and a hair coloring agent. The hair coloring agent is chosen to match the color of the hair fibers of the consumer in order to highlight the color of the hair fibers. Particularly, when the hair coloring composition comprises a hair coloring agent wherein the hair coloring agent preferably comprises one or more colored materials, or one or more pigments, such as metallic oxides, the transmitted light can also reflect on the hair coloring agent, which provides a colored lustre, preferably a metallic lustre for the hair. The colored hair ornament is therefore a sophisticated hair decoration which can highlight the hair style of the consumer but also the color of the natural or dyed hair fibers.

## Light-transmissive filament

The colored hair ornament comprises a light source; and a plurality of light-transmissive filaments. A conventional hair ornament comprises a housing, a power supply coupled to the housing, a light source, such as LED, electrically coupled to the power supply and a plurality of light-transmissive filaments such as optical fibers positioned to receive light from the light source and having fiber ends protruding out and away from the housing.

The housing comprises two pieces, a case with hollow compartments, and a sliding lid, which when closed conceals the compartments, or when open allows access to the compartments. The power supply may comprise one or more batteries to power the light source.

Each light-transmissive filament has a proximal end, a distal end and a side wall. The proximal end of each light-transmissive filament is arranged to receive light from the light source.

Fig. 1 is a schematic diagram of a light-transmissive filament 10 according to the present invention. The light-transmissive filament is coated with a hair coloring composition comprising a film-forming aminosilicone polymer 70 and a hair coloring agent 75 such as a pigment. The light-transmissive filament comprises a core 20 surrounded by a cladding 30. The light-transmissive filament can be a conventional optical fiber. Conventional optical fibers rely on the principle of total internal reflection to transmit light from one end of the fiber to the other. Fig. 1 shows a light ray 60 entering the fiber 10 at the proximal end 40 of the fiber (nearest to the light source) and exiting the fiber at the distal end 50. The difference in refractive index between the core 20 and the cladding 30 ensures that the light is reflected back into the core every time it reaches the core-cladding interface (as long as the ray is initially coupled into the fiber within a

predetermined range of angles). In theory, in a perfect optical fiber, all of the light coupled into the fiber 10 at the proximal end 40 would be carried through the fiber to emerge at the distal end 50.

It would be desirable to create a line of light. A reflective coating may be applied to the distal end of each light-transmissive filament protruding from the light source. The distal ends of the one or more light-transmissive filaments may be cut at a 90-degree angle. The distal end of each light-transmissive filament may be coated with a reflective coating as shown in Fig. 2.

The reflective coating may be a paint with reflective particles that are smaller in size than the diameter of the light-transmissive filaments of the plurality of light-transmissive filaments. Fig. 2 is a schematic diagram of a light-transmissive filament 10 according to the present invention. The distal end of a light-transmissive filament 10 is cut at a 90-degree angle. The distal end 50 of the light-transmissive filament 10 is coated with a reflective coating 80. The light ray 65 traveling axially down the light-transmissive filament is therefore reflected back up the light-transmissive filament 10. The reflected light travels in both the core 20 and the cladding 30 of the light-transmissive filament 10 and thus is scattered sideways and out of the light-transmissive filament 10. The entire length of the light-transmissive filament 10 therefore emanated light and appears as a line of light. The light-transmissive filament is coated with a hair coloring composition comprising a film-forming aminosilicone polymer 70 and a hair coloring agent 75 such as a pigment. The reflected light scattered sideways and out is able to be reflected on the hair coloring agent.

Alternatively, it would be desirable to create a point of light. The distal end protruding out and away from the light source may be cut at an angle to maximize observed brightness of light emitted from the light source. Observed brightness is increased due to the increased surface area of the exit from the distal end of the light-transmissive filament.

The plurality of light-transmissive filaments may be cut at the distal ends at an angle ranging from 15 to 75 degrees, preferably from 30 to 50 degrees, more preferably at an angle of 45 degrees. The angle may range between 15 and 75 degrees, preferably between 30 to 50 degrees, more preferably 45 degrees with respect to the length of the light-transmissive filaments.

Fig. 3 is a schematic diagram of a light-transmissive filament 10 according to the present invention. The distal end 50 of the light-transmissive filament 10 is cut an a 45-degree angle in order to maximize the intensity of light transmitted perpendicular to the fiber length and outwards away from a user towards observers. The remainder of the light 65 travelling axially down the light-transmissive filament 10 that is not reflected outwards perpendicularly is transmitted downward and out the distal end 50. The light-transmissive filament is coated with a hair coloring composition comprising a film-forming aminosilicone polymer 70 and a hair coloring agent 75 such as a pigment. The reflected light transmitted outwards perpendicularly as set out above is also able to be reflected on the hair coloring agent.

Alternatively, it would be desirable to allow some or all of the light to escape through the side wall of the optical fiber. This could allow the creation of a glowing strand or "line of light". Hence, each light-transmissive filament may be configured to emit through the side wall at least some of the light received at the proximal end, the light-transmissive filament comprising a first segment of unit length, located at a first distance from the proximal end, the first segment being configured to emit through the side wall a first proportion of the light arriving at the first segment from the proximal end. The light-transmissive filament may further comprise a second segment of unit length, located at a second distance from the proximal end, the second segment being configured to emit through the side wall a second proportion of the light arriving at the second segment from the proximal end. The first distance may be preferably greater than the second distance. An optical property of the light-transmissive filament may vary over a length of the filament such that the first proportion is greater than the second proportion.

Fig. 4 schematically illustrates a preferred light-transmissive filament 15. The filament 15 may have a proximal end 40 for receiving light from a light source and a distal end 50. A side wall may extend between the two ends. In this example, the filament has a circular cross section and therefore the side wall is approximately cylindrical (when the filament is straight). The side wall of the filament may be configured to emit at least some of the light that is received through the proximal end 40 of the filament. An optical property of the filament may vary over the length of the filament so that it is easier for light to escape from some parts of the filament than others. In greater detail: the light-transmissive filament may comprise a first segment located at a first distance from the proximal end. In this first segment, the filament may be configured to emit a first proportion of the light arriving from the proximal end 40. The filament may further comprise a second segment located at a second distance from the proximal end 40. This second segment may be configured to emit a second proportion of the light arriving from the proximal end. The optical property of the filament may vary such that the first proportion is greater than the second proportion.

By varying the optical property in this way, the amount of light escaping from the filament at any local segment along its length can be controlled. A wide variety of effects can be created.

One advantageous example can be a filament 15 in which the proportion of light escaping increases from the proximal end 40 to the distal end 50. In this case, the first distance is greater than the second distance - that is, the first segment is nearer to the distal end 50 than the second segment is. Preferably, the optical property may vary continuously over the length of the filament. This means that any first segment and any second segment (of the same non-trivial length) will have the relationship that the proportion of light that escapes from the first segment is greater than the second

segment, provided that the first segment is further from the proximal end 40. This is the example considered in Fig. 4. The light-transmissive filament is coated with a hair coloring composition comprising a film-forming aminosilicone polymer 70 and a hair coloring agent 75 such as a pigment. The reflected light escaping from the cladding as set out above is also able to be reflected on the hair coloring agent.

The light-transmissive filament may comprise a core 20 and a cladding 30. The optical property that varies over the length of the filament may comprise a property of the cladding 30. Like the optical fiber 10 of Fig. 1, the light-transmissive filament 15 of Fig. 4 comprises a core 20 and a cladding. The core 20 can be substantially similar to the conventional core of Fig. 1.

The cladding may be a partial cladding 32. In a first segment, closer to the distal end 50, a first area of the core 20 may be exposed by the partial cladding 32. In a second segment, closer to the proximal end 40, a second area of the core 20 may be exposed. The first area may be greater than the second area. This means that the proportion of the surface area of the core that is exposed successively may increase from the proximal end 40 to the distal end 50. In this way, by varying a property of the cladding, a greater proportion of light can be allowed to escape at locations closer to the distal end 50.

Without wishing to be bound by theory, it is believed that the filament 15 functions as follows. In the embodiment illustrated in Fig. 4, light is allowed to escape by eliminating the cladding from localized parts of the core 20. The light ray 60 illustrates the effect of this. Where the ray 60 encounters an interface between the core 20 and the partial cladding 32, it is reflected back into the core 20. However, when the light ray 60 encounters an exposed area 34 it is no longer internally reflected and therefore is emitted from the filament. This is believed to be a result of the change in critical angle caused by the removal of the cladding. Other effects may also contribute to the escape of light. For example, the removal of the cladding may also modify the surface of the core in such a way as to promote the escape of light.

Light is coupled into the core 20 at the proximal end 40 of the filament 15. It travels down the filament by internal reflection until it escapes through the side wall of the filament 15. As more light escapes through the side wall, the amount of light remaining within the filament to continue towards the distal end is reduced. Therefore, the amount of light in the filament decays from the proximal end 40 to the distal end 50. If the exposed areas 34 were uniformly distributed over the length of the filament 15, then each ray of light would have an equal chance of escaping through the side wall in any longitudinal segment of a given size. This would result in the filament 15 glowing brighter nearer to the proximal end 40 and glowing less brightly nearer to the distal end 50, because of the decay of light away from the light source, due to the escaping light. To compensate for this, the exposed area per unit length of the filament 15 is increased over the length of the filament, from the proximal end 40 to the distal end 50. Thus, although there is more light being transmitted through the filament nearer to the proximal end 40, this light has a lower chance of escaping through the side wall. There is proportionally less light propagating in the filament nearer to the distal end 50, but this light has a proportionally greater chance of escaping through the side wall, because of the increasing exposed area 34. This can allow a more uniform brightness to be produced over the length of the filament 15.

The light-transmissive filament 15 may be preferably similar in its basic construction to a polymer optical fiber 10. The light-transmissive filament may comprise a core 20 which is formed of Poly(methyl methacrylate) (PMMA). The light-transmissive filament may comprise a cladding, and/or a partial cladding 32 which is formed of a fluorinated polymer, such as a fluorocarbon. The filament 15 may be fabricated with a maximum overall thickness (maximum overall diameter, for a cylindrical filament) of less than 1 mm, preferably less than 500 $\mu$m, more preferably of less than 200 $\mu$m.

Fig. 5 is a schematic block diagram of a hair ornament incorporating a plurality of filaments 130 like the filaments 10 or 15 shown in Figs. 1-4. The hair ornament 100 comprises a light source 110. In this example, the light source 110 includes one or more Light Emitting Diodes (LEDs). The proximal end 132 of each of the filaments 130 is arranged to receive light from this light source 110. Preferably the filaments are held in a bundle and a lens focuses the light from the light source onto the end of the bundle. The filaments may be held in the bundle by gluing them together at their proximal ends, and/or by crimping them together at their proximal ends with a crimp sleeve or collar.

The hair ornament may comprise a fastening means for securing the hair ornament to a wearer's hair. The fastening means may be selected from the group consisting of a hair barrette, a hair comb, a hair clip and combinations thereof.

Light is emitted through the side wall over the length of each filament 130. Any light that has not escaped through the side wall is emitted from the distal end 134 of each filament 130. The filaments 130 may preferably have lengths in the range 2 cm to 100 cm. They are flexible, allowing them to emulate hair. The hair ornament may further comprise a power source (preferably a battery, not shown in Fig. 3) for powering the light source 110. It may also comprise a controller for controlling the light source. Further details of ways to control the light source of a hair ornament can be found in the co-pending European patent application filed on the same date, by the present applicant, entitled "Hair Ornament, Control Device, and Method of Controlling a Hair Ornament".

Also, details of ways to produce light-transmissive filaments 15 like the one illustrated in Fig. 4 can be found in the co-pending European patent application filed on the same date, by the present applicant, entitled "Light-transmissive filament". A method for producing the light-transmissive filament may comprise providing a light-transmissive filament.

This may be an optical fiber 15 with a core 20 and a cladding 30. The method may then comprise modifying the cladding 30 by removing a portion of it. In this embodiment, the step of modifying the cladding may preferably comprise laser-ablating a portion of the cladding so that an area 34 of the core 20 is exposed, as described already above. Following the laser-ablation, the cladding 30 becomes a partial cladding 32 with exposed areas 34 where the cladding 30 has been removed by the laser, and the optical fiber 10 becomes a light-transmissive filament according to an embodiment of the invention. The laser-ablation may be controlled so that a greater proportion of the cladding is removed in a first segment, closer to the distal end 50, compared with a second segment, closer to the proximal end 40. The proportion of light escaping in a given segment of unit length may vary in direct relation to the proportion of exposed area 34.

The laser power may be preferably selected so that all of the cladding is removed, at the sites where the beam strikes the fiber. In general, it may be difficult to select a laser power that removes all of the cladding while leaving the core completely unscathed. Therefore, in order to ensure that all of the cladding is removed it may be necessary to set the laser power so that a small amount of core material is also removed.

The individual area of cladding removed by each laser strike may be controlled by varying the spot size of the laser. Spot size can be controlled by adjusting the focus of the laser beam. The total area of cladding removed can be controlled by controlling either the size of the individual areas, or the spacing between exposed areas, or a combination of both. Using a smaller spot size and a larger number of individual areas can enable finer control of the amount of cladding removed in any given longitudinal segment of the filament.

The laser may be controlled to create "scratches" spaced at intervals along the length of each filament. Each scratch may be formed by one or more scribe lines created by laser ablation. Each scribe line may correspond to one pass of the laser. In one example, the spot size of the laser is 30 μm in diameter. The number of scribe lines per scratch is increased towards the distal end of the filament. This increases the proportion of exposed area towards the distal end. The scratches may be evenly or unevenly spaced over the length of the filament.

Alternatively to the laser beam, a chemical treatment may be used. The step of modifying the cladding may comprise chemically treating the cladding to damage it, such that the cladding has a first degree of damage in the first segment and a second degree of damage in the second segment, the first degree of damage being greater than the second degree of damage.

It is possible to produce light-transmissive filaments with desirable properties according to embodiments of the invention by other alternative methods.

For example, the optical property that varies over the length of the filament may comprise a surface-roughness of the filament. This may be appropriate for a filament that comprises a core with no cladding. The proportion of light escaping through the side wall of the filament will vary in direct relation to the roughness of the surface of the filament. Without wishing to be bound by theory, it is believed that increasing the surface roughness has the effect of creating many small facets in the surface of the filament, at different angles. This increases the range of angles for which light propagating inside the filament is able to escape from it. Therefore, by increasing the surface-roughness from the proximal end 40 to the distal end 50 a more uniform brightness of the side wall of the filament may be obtained. Surface roughness can be measured using conventional methods. For example, it is known to measure surface roughness using a roughness tester comprising a thin needle or stylus. The tip of the stylus is mechanically drawn across the surface and the movement of the stylus is measured to determine roughness. This method can be used to measure roughness even at a very fine scale. For example, it is known to use a very thin stylus to measure the roughness of semiconductor or Micro-Electro-Mechanical System (MEMS) devices. The same technique can be used to measure the roughness of a filament according to an embodiment. The surface-roughness may be adjusted by chemical or mechanical treatment of the filament, or by laser ablation using a suitable laser power.

A further alternative for filaments having a core 20 and cladding 30 may be to vary the refractive index of the cladding in the longitudinal direction (that is, along the length of the filament). The refractive index of the cladding determines the range of angles of incidence at which light is reflected internally back into the core 20. Therefore, by varying the refractive index of the cladding 30, light reaching the core-cladding interface at varying angles of incidence can be selectively permitted to escape through the side wall. Longitudinal variation of the refractive index of the cladding 30 may be possible by doping the cladding such that the dopant concentration varies along the length of the fiber. For example, adding germanium can increase the refractive index, while adding fluorine can reduce it. The refractive index of doped material can be determined by the linear relationship between the doped material's mole percentage and permittivity. Assume that $n_0$ is the refractive index of the host material and $n_1$ is the refractive index of $m_1$ mole-percentage doped material. Then, the refractive index n of m mole-percentage doped material can be interpolated as:

$$n^2 = n_0^2 + \frac{m}{m1}(n_1^2 - n_0^2)$$

Among the methods discussed above, laser-ablation is currently preferred. It allows precise control of the areas

34 of cladding that are removed to expose the core 20. Both the size and position of the exposed areas 34 can therefore be controlled accurately. Chemically treating the cladding can be faster and cheaper than laser-ablation, but it can be more difficult to accurately control the degree of damage to the cladding 30 over the length of the filament. The approach of using surface-roughness to control the escape of light may have the disadvantage that surface-roughness can be modified accidentally during use, for example by abrasion of the filament. Modifying the refractive index longitudinally may be costly.

The overall thickness (diameter) of the filaments may vary over the longitudinal dimension. For example, one or more filaments may have a diameter increasing from the proximal end to the distal end. This can emulate real hair, in which the individual strands of hair are thicker at the ends.

Another possibility is that the filament may comprise a layer of keratin or a layer of melanin, or both. The keratin or melanin may be the outermost layer of the filament. In one case, the keratin or melanin may act as the cladding of the filament. In another case, the keratin or melanin may be an additional layer coated over the cladding of the filament. Keratin and melanin are constituents of natural hair. Therefore, incorporating a layer of keratin and/or melanin in the filament can give the filament a more natural appearance, like real hair, thus mimicking hair. When the filament is not illuminated, it may be less obtrusive in appearance, because its appearance is similar to the strands of hair on the wearers head. And when the filament is illuminated, it may appear as though the wearer’s hair itself is glowing. Melanin is a pigment. Therefore, the amount or concentration of melanin may be used to give the filament a desired color. In particular, it can be used to color the filament to even more match the color of a wearer’s hair.

Hair coloring composition

A hair coloring composition for providing a film on the plurality of light-transmissive filaments is provided. Indeed, the combination of a film-forming aminosilicone polymer as set out more in detail hereinafter, and a hair coloring agent, optionally in a cosmetically acceptable carrier results in a film on the plurality of light-transmissive filaments, and optionally on the hair fibers, that is sufficiently durable that it does not quickly rub off whilst on the plurality of light-transmissive filaments, for example on the hands, clothes etc of the consumer. Indeed, the film-forming aminosilicone polymer enables the forming of a very thin, dry film, which tightly binds any hair coloring agent, e.g. pigment and/or colored material onto the plurality of light-transmissive filaments in a durable fashion. Hence, the film-forming aminosilicone polymer provides an excellent film.

The provision of the film-forming aminosilicone polymer in the hair coloring composition can help to provide an excellent support system for any type of hair coloring agent such as pigment and/or colored material, which can provide the desired effects to the colored hair ornament, such as colored lustre, preferably a metallic lustre for the hair. The colored hair ornament is therefore a sophisticated hair decoration which is able to highlight the hair style but also the color of the natural hair fibers and/or the color of the dyed hair fibers. It can also modify the color of the light emitted through the filaments, because the pigment acts as a filter, selectively transmitting predetermined wavelengths of light. Because of this effect, the color of the surrounded hair fibers can be further highlighted.

The hair coloring composition as set out herein above is for providing a film on the plurality of light-transmissive filaments and optionally the keratin fibers. The hair coloring composition may be for providing a film, preferably comprising pigment or colored material on keratin fibers. The film may be a durable film.

The hair coloring composition may be substantially free of compounds causing precipitation of any component of the hair coloring composition when the hair coloring composition is in aqueous solution at pH 5 and at 23°C.

Indeed, precipitation of any component of the present composition would have negative side effects on the efficacy of the present invention - particularly in terms of hair feel. Indeed, with the exclusion of the hair coloring agent that is herein intended to be immobilised on the plurality of light-transmissive filaments, other residues on hair are not accepted by the consumer. For example, precipitation of the film-forming aminosilicone polymer would detract from their ability to form a film on the light-transmissive filaments and would form residues on the colored hair ornament and hair. The hair coloring composition may be substantially free of any further ionic compounds. The hair coloring composition may be substantially free of any compounds that are cationic at pH 4.0 to 5.0.

Cosmetically acceptable carrier

The hair coloring composition may comprise a cosmetically acceptable carrier. The cosmetically acceptable carrier of the hair coloring composition may be an aqueous carrier. The hair coloring composition may comprise water. Water can provide a hydrophilic phase, which the hydrophilic portions of any other ingredients comprised in the hair coloring composition can interact with water. Water can also provide a fluid phase meaning that the hair coloring composition can be in liquid form. The hair coloring composition may comprise from 50% to 85% water, or from 65% to 75% of water by total weight of the composition.

The cosmetically acceptable carrier may be any carrier suitable for formulating the hair coloring composition

being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the composition. The alcohol of the hair coloring composition may be selected from the group consisting of ethanol, isopropanol, propanol, and mixtures thereof.

When the cosmetically acceptable carrier is an aqueous carrier, the aqueous carrier may consist essentially of water and may be substantially free of alcohol. The hair coloring composition may comprise a safe and effective amount of a cosmetically acceptable carrier which is water. The hair coloring composition may comprise from 0.1% to 99%, or from 1% to 98%, or from 10% to 97%, or from 30% to 95% of water by total weight of the composition.

When used daily, organic solvents in the hair coloring compositions may have a negative effect on humans and on the environment. They also have an unpleasant odor. Hence, the hair coloring composition may be substantially free of organic solvents. Organic solvents may be any volatile alcohols, e.g. ethanol, isopropanol, propanol.

The hair coloring composition may be substantially free of alcohol, such as volatile alcohols (e.g. ethanol, isopropanol, propanol). When the hair coloring composition is substantially free of alcohol, the hair coloring composition can advantageously have a reduced odour. Flammability issues can also be prevented.

The cosmetically acceptable carrier of the hair coloring composition may be an oily compound. The oily compound may be selected from the group consisting of cyclic silicones and volatile hydrocarbons. Cyclic silicones can be available from Dow Corning. The cyclic silicone may have from at least 3 silicone atoms or from at least 5 silicone atoms but no more than 7 silicone atoms or no more than 6 silicone atoms. The cyclic silicone may conform to the formula:

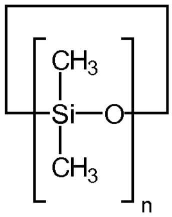

wherein n is from 3 or from 5 but no more than 7 or no more than 6. The cyclic silicone may have a kinematic viscosity of less than 10 cPs at 23°C. A Suitable cyclic silicone for use herein may include Cyclomethicone D5 (commercially available from G.E. Silicones). Alternatively, the hair coloring composition may be silicone-free.

Volatile hydrocarbons e.g. Isopar can be obtained from ExxonMobil Petroleum and Chemical. The oily compound may be a mineral oil. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

Film-forming aminosilicone polymer

The hair coloring composition comprises a film-forming aminosilicone polymer, wherein the film-forming aminosilicone polymer comprises an aminosilicone polymer and a silicone resin. The aminosilicone polymer comprises amino side chains, and wherein the aminosilicone polymer has a weight average molecular weight of from 10,000 Dalton to 60,000 Dalton. "Sidechain" (aka "side chain") in the context of a silicone refers to a group being not part of the silicone backbone nor only present on at least one terminus of the silicone backbone. "Terminal aminosilicone" as defined herein means silicone comprising one or more amino groups at one or both ends of the silicone backbone. Aminosilicone polymers having amino side chains are sometimes referred to as silicone compounds comprising pendant amino groups. The aminosilicone polymer may be not a terminal aminosilicone. The hair coloring composition may be substantially free of silicones having terminal amino groups.

The aminosilicone polymer is a film-forming aminosilicone polymer. The aminosilicone polymer may be a polydimethylsiloxane having graft amino groups.

The aminosilicone polymer may have a weight average molecular weight of from 15,000 Dalton to 50,000 Dalton, or from 20,000 Dalton to 40,000 Dalton.

The aminosilicone polymer may be a polydimethylsiloxane polymer having pendent (graft) amino groups. The aminosilicone polymer may conform to the formula:

...in which x' and y' are integers such that the weight average molecular weight is between 10,000 Dalton and 60,000 Dalton. The endcaps may be methoxy rather than hydroxyl as pictured

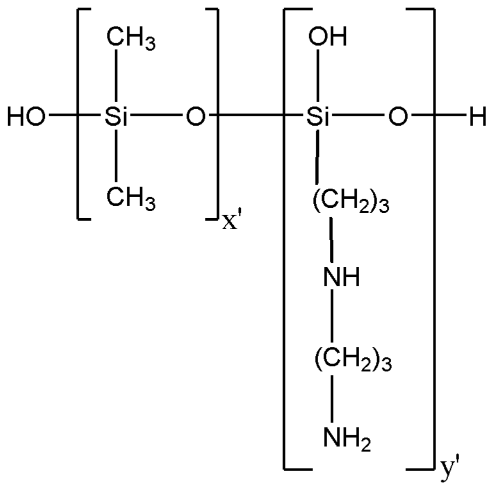

in the above formula.

The aminosilicone polymer may be a polydimethylsiloxane polymer having a sidechain with from 3 to 8 carbon atoms. The sidechain may comprise carbon, hydrogen and nitrogen atoms. The sidechain may consist of carbon, hydrogen and nitrogen atoms. The aminosilicone polymer may be a polydimethylsiloxane polymer having an aminoethyl aminopropyl sidechain.

The aminosilicone polymer may conform to the formula:

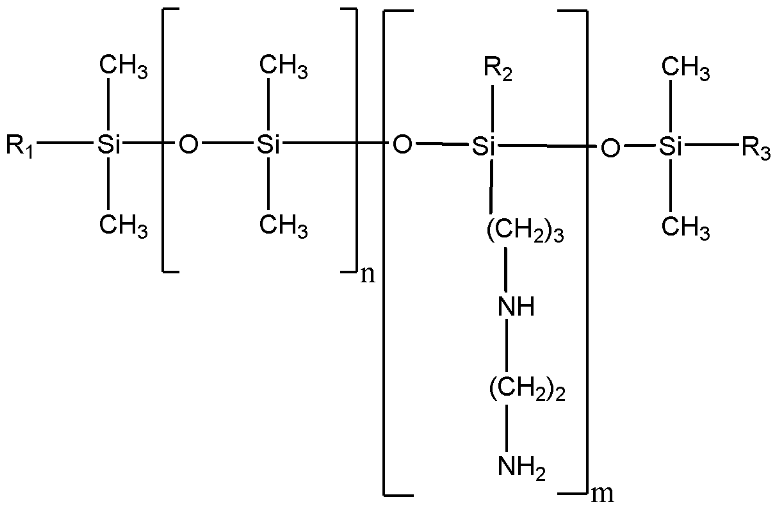

...in which n and m are integers such that the weight average molecular weight is between 10,000 Dalton and 60,000 Dalton, $R_1$ and $R_3$ are independently selected from $-OH$ or $-OCH_3$; $R_2$ is H or a $C_1$ to $C_3$ alkyl, or methyl or H, preferably methyl. "n" may be on average from 1 to 50, or from 5 to 20, or from 6 to 10, or from 8 to 9, and "m" may be on average from 120 to 300, or from 150 to 200. "n" may be on average from 5 to 8, "m" may be on average from 150 to 180, $R_1$ and $R_3$ may be both methyl, and $R_3$ may be $-OCH_3$.

The aminosilicone polymer may have an amine number of from 0.1 meq/g to 3 meq/g, or from 0.7 meq/g to 2.5 meq/g, or from 0.6 meq/g to 1 meq/g.

Suitable example aminosilicone polymers can be found in the following patent documents: Decoster US6,451,747B1 col. 17, 1.4-27; Hughes US5,567,428 col.13, 1.40-56; Gawtrey et al US2004/0010863A1, §0016 to §0039; Mahr et al US2006/0041026A1.

The hair coloring composition may comprise from 1% to 15%, or from 1.5% to 5% of an aminosilicone polymer by total weight of the composition. The viscosity of the aminosilicone polymer may be from 10 to 100,000 mPa·s, or from 100 to 10,000 mPa·s.

The hair coloring composition comprises a silicone resin. Silicone resins are known in the art. The silicone resin may be a film-forming polymer. The silicone resin may be preferably an MQ resin. "M" stands for $Me_3SiO$ and "Q" stands for $SiO_4$. The MQ resin may have an M:Q molar ratio of from 0.5:1.0 to 1.5:1.0. The weight average molecular weight

of the resin may be from 1000 Daltons to 10,000 Daltons. The MQ resin may contain at least 80 mol.%, or at least 95 mol.%, of units of the general formula below:

$R^7{}_3SiO_{1/2}$

$SiO_{4/2}$

in which $R^7$ is $C_{1-40}$ alkyl, H, -OR or -OH radicals. The ratio of the units of the general formulae may be from 0.5 to 2.0, or from 0.5 to 1.5. The not more than 3% by weight, or not more than 2.5% by weight, of the radicals $R^7$ may be -OR and -OH.

The remaining units of the MQ silicone resin may be units of the following general formulae:

$R^7{}_2SiO_{2/2}$

$R^7SiO_{3/2}$

in which $R^7$ is $C_{1-40}$ alkyl, H, -OR or -OH radicals.

$R^7$ may be $C_{1-40}$ alkyl that is optionally halogen-substituted, linear, cyclic, branched, aromatic, saturated or unsaturated. $R^7$ may be an alkyl group having $C_{1-6}$ carbon atoms, or a phenyl radical. The halogen substituents may be selected from fluorine and chlorine. $R^7$ may be selected from methyl, ethyl, phenyl and H. The hair coloring composition may comprise from 0.1% to 10%, or from 1% to 5%, or from 2% to 4% of a MQ resin.

MQ resins are available from Wacker-Chemie AG, D-81737 München, Germany. For example, MQ-RESIN POWDER 803 TF is a co-hydrolysis product of tetraalkoxy silane (Q unit) and trimethyl-ethoxy silane (M unit) and can be seen as a three dimensional network of polysilicic acid units which are endblocked with trimethylsilyl groups. Some residual ethoxy and hydroxy functions are present. MQ resins are also available from Dow Corning. For example, Dow Corning® MQ-1640 Flake Resin is a combination of MQ and T propyl silicone resin and has the INCI name: Trimethylsiloxy silicate (and) Polypropyl silsesquioxane.

The hair coloring composition may comprise an ether of a water-soluble polyhydric alcohol. The ether of a water-soluble polyhydric alcohol has the advantage that it is able to prevent the aminosilicone and the silicone resin from forming a complex. The ether of a water-soluble polyhydric alcohol may be a non-polymeric, amphiphilic compound. Indeed, the aminosilicone comprises amino side chains, which lend hydrophilic character to the aminosilicone, and the silicone resin typically is hydrophobic in nature. Thus where the ether of a water-soluble polyhydric alcohol has amphiphilic chemistry it can interact with both the aminosilicone and the silicone resin and keep them from clumping, and also from precipitating. The hair coloring composition may comprise ether of a water-soluble polyhydric alcohol, wherein the ether of a water-soluble polyhydric alcohol is selected from the group consisting of diethyleneglycol monobutylether, ethylene glycol monohexyl ether, and a mixture of diethyleneglycol monobutylether and ethylene glycol monohexyl ether. The hair coloring composition may comprise from 0.01% to 20%, or from 0.1% to 10%, or from 0.5% to 5%, or from 1.0% to 5%, or from 2% to 5% ether of a water-soluble polyhydric alcohol by total weight of the composition.

A suitable product for use in the present invention is available under the trade mark Wacker® / BELSIL ADM 8301 E by the company Wacker-Chemie AG, D-81737 München, Germany. This product contains from 10% to 20% of poly[3-((2-aminoethyl)amino)propyl]methyl(dimethyl)siloxane, hydroxyterminated, which is an aminosilicone. It also contains from 0.1% to 0.2% octamethylcyclotetrasiloxane and from 1% to 5% of an MQ silicone resin. The product also contains from 1% to 3% ethylene glycol monohexyl ether and from 5% to 10% diethyleneglycol monobutylether. Said product is described in US2006/0041026A1. A similar product is Wacker® HC303 also from Wacker-Chemie AG.

Alternatively, the film-forming aminosilicone polymer of the present invention may be described in European Patent EP 2 214 633 B1 in par. 11-12.

The film-forming aminosilicone polymer may be the product of the reaction of a siloxane and an amino silane.

The siloxane may comprise at least two oxirane or oxetane groups having the formula:

$$M_fM^E{}_hM^{PE}{}_iM^H{}_jD_kD^E{}_lD^{PE}{}_mD^H{}_nT_oT^E{}_pT^{PE}{}_qT^H{}_qT^H{}_rQ_s$$

with

$M = R^9R^{10}R^{11}SiO_{1/2}$;
$M^H = R^{12}R^{13}HSiO_{1/2}$;
$M^{PE} = R^{12}R^{13}(-CH_2CH(R^{14})(R^{15})_tO(R^{16})_u(C_2H_4O)_v(C_3H_6O)_w(C_4H_8O)_xR^{17})SiO_{1/2}$;
$M^E = R^{12}R^{13}(R^E)SiO_{1/2}$;
$D = R^{18}R^{19}SiO_{2/2}$; and
$D^H = R^{20}HSiO_{2/2}$;

$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17})SiO_{3/2}$;
$T^E = R^ESiO_{3/2}$; and
$Q = SiO_{4/2}$; where
$R^9$, $R^{10}$, $R^{11}$, $R^{12}$, $R^{13}$, $R^{18}$, $R^{19}$, $R^{20}$, and $R^{21}$ are each independently selected from the group of monovalent hydrocarbon radicals having from 1 to 60 carbon atoms;
$R^{14}$ is H or a 1 to 6 carbon atom alkyl group; $R^{15}$ is a divalent alkyl radical of 1 to 6 carbons; $R^{16}$ is selected from the group of divalent radicals consisting of $-C_2H_4O-$, $-C_3H_6O-$, and $-C_4H_8O-$; $R^{17}$ is selected from the group consisting of H, monofunctional hydrocarbon radicals of 1 to 6 carbons, and acetyl;
$R^E$ is independently a monovalent hydrocarbon radical containing one or more oxirane or oxetane moieties having from 1 to 60 carbon atoms;
the subscript f is 0 or positive subject to the limitation that when the subscript f is 0, h must be positive;
the subscript h is 0 or positive subject to the limitations that when h is 0, the subscript f is positive, and that the sum of the subscripts h, 1 and p is positive;
the subscript k is zero or positive and has a value ranging from 0 to 1000;
the subscript 1 is 0 or positive and has a value ranging from 0 to 400 subject to the limitation that the sum of the subscripts h, 1 and p is positive;
the subscript o is 0 or positive and has a value ranging from 0 to 50;
the subscript p is 0 or positive and has a value ranging from 0 to 30 subject to the limitation that the sum of the subscripts h, 1 and p is positive;
the subscript s is 0 or positive and has a value ranging from 0 to 20;
the subscript i is 0 or positive and has a value ranging from 0 to 20;
the subscript m is 0 or positive and has a value ranging from 0 to 200;
the subscript q is 0 or positive and has a value ranging from 0 to 30;
the subscript j is 0 or positive and has a value ranging from 0 to 2;
the subscript n is 0 or positive and has a value ranging from 0 to 20;
the subscript r is 0 or positive and has a value ranging from 0 to 30;
the subscript t is 0 or 1;
the subscript u is 0 or 1;
the subscript v is 0 or positive and has a value ranging from 0 to 100 subject to the limitation that $(v+w+x) > 0$;
the subscript w is 0 or positive and has a value ranging from 0 to 100 subject to the limitation that $(v+w+x) > 0$; and
the subscript x is 0 or positive and has a value ranging from 0 to 100 subject to the limitation that $(v + w + x) > 0$.

Alternatively, the siloxane may be an oxirane or oxetane compound which is a hydrocarbon having the formula:

$$R^{22}_y(R^{23})_z(R^{24}_\alpha)(R^{25})_\beta$$

where $R^{22}$ and $R^{25}$ are independently a monovalent hydrocarbon radical containing one or more oxirane or oxetane moieties having from 3 to 12 carbon atoms;
$R^{23}$ and $R^{24}$ are each selected from the group consisting of H or a linear or branched monovalent hydrocarbon radical of 1 to 200 carbons;
the subscripts y, z, $\alpha$, $\beta$ are zero or positive ranging from zero to four subject to the limitation that $(y + \beta) > 2$ or alternatively where the oxirane or oxetane compound is a polyether having the formula:

$$R^{26}O(R^{27})_\gamma(C_2H_4O)_\delta(C_3H_6O)_\varepsilon(C_4H_8O)_\zeta R^{28}$$

where $R^{26}$ and $R^{28}$ are independently a monovalent hydrocarbon radical containing one or more oxirane or oxetane moieties having from 3 to 12 carbon atoms;
$R^{27}$ is selected from the group of divalent radicals consisting of $-C_2H_4O-$, $-C_3H_6O-$, and $-C_4H_8O-$; the subscript $\gamma$ is zero or 1;
the subscript $\delta$ is zero or positive and has a value ranging from 0 to 100 subject to the limitation that $(\delta + \varepsilon + \zeta) > 0$;
the subscript $\varepsilon$ is zero or positive and has a value ranging from 0 to 100 subject to the limitation that $(\delta + \varepsilon + \zeta) > 0$;
the subscript $\zeta$ is zero or positive and has a value ranging from 0 to 100 subject to the limitation that $(\delta + \varepsilon + \zeta) > 0$.

The aminosilane may have the formula:

$$N(H)(R^1)R^2Si(OR^3)_{3-a-b-c}(OR^4)_a(R^5Si(OR^6)_d(R^7)_e)_bR^8_c$$

with

$R^1$ is chosen from the group consisting of H or a monovalent hydrocarbon radical containing 1 to 20 carbon atoms;
$R^2$ is selected from a group consisting of a divalent linear or branched hydrocarbon radical consisting of 1 to 60 carbons;
$R^4$ is a hydrocarbon radical that contains 3 to 200 carbon atoms;
$R^5$ is selected from a group consisting of oxygen or a divalent linear or branched hydrocarbon radical consisting of 1 to 60 carbons;
$R^3$, $R^6$, $R^7$, and $R^8$ and are each independently selected from the group of monovalent linear or branched hydrocarbon radicals having from 1 to 200 carbon atoms;
the subscript b is 0 or a positive number and has a value ranging from 0 to 3;
the subscript a is a positive number less than 3;
the subscripts b and c are 0 or positive and have a value ranging from 0 to 3 subject to the limitation that $(a+b+c) \leq 3$; and
the subscripts d and e are 0 or positive and have a value ranging from 0 to 3 subject to the limitation that $(d+e) = 3$.

The film-forming aminosilicone polymer of the hair coloring composition may be the product of the reaction of the siloxane (a1), the aminosilane (b1), and a compound (I) having the formula:

$$\text{(I)} \qquad R^{29}(R^{30})_\kappa Si(OR^{31})_{3-\eta-\theta}(R^{32})_\eta(OR^{33})_\theta$$

where

$R^{29}$ is a monovalent hydrocarbon radical containing one or more oxirane or oxetane moieties having from 3 to 12 carbon atoms;
$R^{30}$ is a divalent hydrocarbon radical consisting of 1 to 60 carbons and the subscript $\kappa$ has a value of 0 or 1; $R^{31}$ and $R^{32}$ are independently selected from the group of monovalent linear or branched hydrocarbon radicals having from 1 to 60 carbon atoms;
the subscript $\eta$ is zero or positive and has a value ranging from 0 to 3;
the subscript $\theta$ is greater than 0 and less than or equal to 3, subject to the limitation that $3-\eta-\theta$ is greater than or equal to 0; and
$R^{33}$ is a hydrocarbon radical that contains 3 to 200 carbon atoms.
As used herein the phrase hydrocarbon radical includes hydrocarbon radicals that may be optionally substituted with hetero-atoms particularly nitrogen, oxygen, and sulfur, and may optionally contain ring structures such as oxirane and oxetane groups.

The hair coloring composition may comprise:

the aminosilane wherein $R^1$ has from 1 to 5 carbon atoms; $R^2$ has from 2 to 8 carbon atoms; $R^4$ has from 3 to 8 carbon atoms; $R^3$, $R^6$, $R^7$, and $R^8$ each independently have from 1 to 15 carbon atoms; the subscript a ranges from 2 to 3; the subscript b ranges from 0 to 15; the subscript c ranges from 0 to 2;
the siloxane wherein $R^9$, $R^{10}$, $R^{11}$, $R^{12}$, $R^{13}$, $R^{18}$, $R^{19}$, $R^{20}$, and $R^{21}$ each independently have from 1 to 3 carbon atoms; the subscript k ranges from 5 to 250; the subscripts v, w, and x each independently range from 0 to 35; and
the compound (I) wherein $R^{31}$ and $R^{32}$ each independently have from 1 to 8 carbon atoms, and $R^{33}$ has from 3 to 50 carbon atoms.

The hair coloring composition may comprise:

the aminosilane wherein $R^1$ is H; $R^2$ has from 2 to 5 carbon atoms; $R^4$ has from 3 to 5 carbon atoms; $R^3$, $R^6$, $R^7$, and $R^8$ each independently have from 2 to 8 carbon atoms; the subscript a ranges from 0 or 1; the subscript b is 3; the subscript c is 0 or 1;
the siloxane wherein $R^9$, $R^{10}$, $R^{11}$, $R^{12}$, $R^{13}$, $R^{18}$, $R^{19}$, $R^{20}$, and $R^{21}$ are each independently methyl; the subscript k ranges from 5 to 150; the subscripts v, w, and x each independently range from 0 to 25; and
the compound (I) wherein $R^{31}$ and $R^{32}$ each independently have from 1 to 4 carbon atoms; and $R^{33}$ has from 3 to 10 carbon atoms.

The hair coloring composition may preferably comprise:

the aminosilane wherein $R^1$ is H; $R^2$ has from 2 to 5 carbon atoms; $R^3$ has from 3 to 5 carbon atoms; $R^4$ has from 3 to 5 carbon atoms; $R^3$, $R^6$, $R^7$, and $R^8$ each independently have from 2 to 8 carbon atoms; the subscript a is 1; the subscript b is 0; the subscript c is 0; and
the siloxane wherein $R^9$, $R^{10}$, $R^{11}$, $R^{12}$, $R^{13}$, $R^{18}$, $R^{19}$, $R^{20}$, and $R^{21}$ are each independently methyl; the subscript k ranges from 5 to 150; the subscripts v, w, and x each independently range from 0 to 25; and the subscript n is 0.

The hair coloring composition may more preferably comprise the siloxane which is an epoxy encapped polysiloxane with the average structure:

$$CH_2(O)CHCH_2O(CH_2)_\lambda Si(CH_3)_2O[Si(CH_3)_2O]_kSi(CH_3)_2CH_2CH_2CH_2OCH_2CH(O)CH_2$$

with the subscript k ranges from 5 to 250, from 5 to 150, preferably from 50 to 100, and the subscript $\lambda$ is 2 or 3; and the aminosilane which has the formula $H_2NR^2Si(OR^3)_2(OR^4)$ with
$R^2$ has from 2 to 5 carbon atoms;
$R^3$ has from 3 to 5 carbon atoms; and
$R^4$ has from 3 to 5 carbon atoms.

The hair coloring composition may even more preferably comprise as the siloxane a glycidoxypropyl-terminated dimethyl siloxane polymer with the average structure $CH_2(O)CHCH_2O(CH_2)_3Si(CH_3)_2O[Si(CH_3)_2O]_{50}Si(CH_3)_2CH_2CH_2CH_2OCH_2CH(O)CH_2$, and as the aminosilane the aminopropyltriisopropoxysilane.

The hair coloring composition may even more preferably comprise the film-forming aminosilicone polymer which is the product of the reaction of the siloxane (a1), the aminosilane (b1), and an encapped polyether with the average structure $CH_2(O)CHCH_2O(CH_2CH_2O)_\mu CH_2CH(O)CH_2$ with the subscript $\beta$ ranges from 10 to 20, or an encapped polyether with the average structure $CH_2(O)CHCH_2O(CH_2CH_2O)_\mu(CH_2CHCH_3O)_vCH_2CH(O)CH_2$ with the subscript $\mu$ ranges from 1 to 20 and with the subscript v ranges from 1 to 20.

The hair coloring composition may even more preferably comprise as the siloxane a glycidoxypropyl-terminated dimethyl siloxane polymer which is the epoxy encapped polysiloxane with the average structure $CH_2(O)CHCH_2O(CH_2)_3Si(CH_3)_2O[Si(CH_3)_2O]_{50}Si(CH_3)_2CH_2CH_2CH_2OCH_2CH(O)CH_2$, and as the aminosilane the aminopropyltriisopropoxysilane, and as the encapped polyether PEG-13 diglycidyl ether, and optionally diethylaminopropylamine.

In reacting the siloxane (oxirane or oxetane) compounds with amino bearing compounds, the mole ratio of oxirane or epoxy groups to amino groups may be preferably 1 to 4, more preferably greater than 1.1 and less than 3.9, and most preferably greater than 1.2 and less than 3.8. $R^1$ may be preferably a monovalent hydrocarbon radical of from 1 to 10 carbon atoms or hydrogen, more preferably from 1 to 5 carbon atoms or hydrogen, most preferably $R^1$ may be H. $R^2$ may be preferably a monovalent hydrocarbon radical of from 1 to 10 carbon atoms more preferably 2 to 8 carbon atoms, and most preferably 3 to 5 carbon atoms. $R^4$ may be preferably a monovalent hydrocarbon radical of from 3 to 10 carbon atoms, more preferably 3 to 8 carbon atoms, most preferably 3 to 5 carbon atoms. $R^3$, $R^6$, $R^7$, and $R^8$ may be each preferably a monovalent hydrocarbon radical of from 1 to 20 carbon atoms, more preferably 1 to 15 carbon atoms, most preferably 2 to 8 carbon atoms. Subscript a may be in the range of from 0 to 3, preferably from 1 to 3, more preferably from 2 to 3, most preferably from 0 to 1. Subscript b may be in the range of 0 to 25, more preferably 0 to 15 and most preferably 3. Subscript c may be in the range 0 to 3, more preferably 0 to 2, most preferably 0 to 1. $R^9$, $R^{10}$, $R^{11}$, $R^{12}$, $R^{13}$, $R^{18}$, $R^{19}$, $R^{20}$, and $R^{21}$ may be each preferably a monovalent hydrocarbon radical of from 1 to 4 carbon atoms, more preferably 1 to 3 carbon atoms, and most preferably 1 carbon atom. The subscripts f, 1, m, n, o, p, q, r, s may be each in the range of 0 to 200, more preferably 0 to 100, and most preferably 0 to 50. The subscript k may be in the range of 0 to 500, more preferably 5 to 250, and most preferably 5 to 150. The subscripts v, w, and x may be each in the range of 0 to 50, more preferably 0 to 35, and most preferably 0 to 25. $R^{23}$ and $R^{24}$ may be each preferably a monovalent hydrocarbon radical of from 5 to 1000 carbon atoms, more preferably 10 to 500, and most preferably 10 to 300. The subscripts $\delta$, $\varepsilon$, $\zeta$ may be in the range of 0 to 50 more preferably, 0 to 30, and most preferably 0 to 15. $R^{31}$ and $R^{32}$ may be each preferably a monovalent hydrocarbon radical of from 1 to 10 carbon atoms, more preferably 1 to 8 carbon atoms, and most preferably 1 to 4 carbon atoms. $R^{33}$ may be each preferably a monovalent hydrocarbon radical of from 3 to 100 carbon atoms, more preferably 3 to 50 carbon atoms, most preferably 3 to 10 carbon atoms.

Reference is made to substances, components, or ingredients in existence at the time just before first contacted, formed in situ, blended, or mixed with one or more other substances, components, or ingredients in accordance with the present disclosure. A substance, component or ingredient identified as a reaction product, resulting mixture, or the

like may gain an identity, property, or character through a chemical reaction or transformation during the course of contacting, in situ formation, blending, or mixing operation if conducted in accordance with this disclosure with the application of common sense and the ordinary skill of one in the relevant art (e.g., chemist). The transformation of chemical reactants or starting materials to chemical products or final materials is a continually evolving process, independent of the speed at which it occurs. Accordingly, as such a transformative process is in progress there may be a mix of starting and final materials, as well as intermediate species that may be, depending on their kinetic lifetime, easy or difficult to detect with current analytical techniques known to those of ordinary skill in the art.

Reactants and components referred to by chemical name or formula in the specification or claims hereof, whether referred to in the singular or plural, may be identified as they exist prior to coming into contact with another substance referred to by chemical name or chemical type (e.g., another reactant or a solvent). Preliminary and/or transitional chemical changes, transformations, or reactions, if any, that take place in the resulting mixture, solution, or reaction medium may be identified as intermediate species, master batches, and the like, and may have utility distinct from the utility of the reaction product or final material. Other subsequent changes, transformations, or reactions may result from bringing the specified reactants and/or components together under the conditions called for pursuant to this disclosure. In these other subsequent changes, transformations, or reactions the reactants, ingredients, or the components to be brought together may identify or indicate the reaction product or final material.

In describing the products of the instant invention as a reaction product of initial materials reference is made to the initial species recited and it is to be noted that additional materials may be added to the initial mixture of synthetic precursors. These additional materials may be reactive or non-reactive. The defining characteristic of the instant invention is that the reaction product is obtained from the reaction of at least the components listed as disclosed. Non-reactive components may be added to the reaction mixture as diluents or to impart additional properties unrelated to the properties of the composition prepared as a reaction product. Additional reactive components may also be added; such components may react with the initial reactants or they may react with the reaction product; the phrase "reaction product" is intended to include those possibilities as well as including the addition of non-reactive components.

Optionally, the film-forming aminosilicone polymer of the hair coloring composition may be the product of the reaction of the siloxane and the aminosilane in the presence of a primary or a secondary amine.

The primary amine may be preferably selected from the group consisting of, methylamine, ethylamine, propylamine, ethanol amine, isopropylamine, butylamine, isobutylamine, hexylamine, dodecylamine, oleylamine, aniline aminopropyltrimethylsilane, aminopropyltriethylsilane, aminomorpholine, 3-(diethylamino)propylamine, benzylamine, napthylamine 3-amino-9-ethylcarbazole, 1 -amino heptaphlorohexane, 2,2,3,3,4,4,5,5,6,6,7,7,8,8,8-pentadecafluoro-1-octanamine, and mixtures thereof. The primary amine may be more preferably 3-(diethylamino)propylamine.

Alternatively, the secondary amine may be selected from the group consisting of methylethylamine, methylhexylamine, methyloctadecylamine, diethanolamine, dibenzylamine, dihexylamine, dicyclohexylamine, piperidine, pyrrolidine, phthalimide, and mixtures thereof.

Suitable example film-forming aminosilicone polymers can be found in the following European patent EP 2 214 633 B1 in par. 40-51.

The hair coloring composition may comprise from 1% to 20%, preferably from 1.5% to 15%, more preferably from 5% to 10% of a film-forming aminosilicone polymer, by total weight of the composition.

A suitable film-forming aminosilicone polymer for use in the present invention is available under the trade mark Silsoft® CLX-E by the company Momentive Performance Materials, Albany, New York, (US). Silsoft® CLX-E has as the INCI name "dipropylene glycol and polysilicone-19". Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane.

<u>Hair coloring agent</u>

The hair coloring composition comprises a hair coloring agent. The hair coloring agent may be selected from the group consisting of pigment, colored material, and mixtures thereof.

Pigments

The hair coloring agent may comprise one or more pigments, preferably may consist essentially of one or more pigments. The one or more pigments may be one or more colored pigments which impart color effects to the colored hair ornament. Alternatively, the one or more pigments may be lustre effect pigments which impart desirable and aesthetically pleasing lustre effects to the hair coloring composition or to the plurality of light-transmissive filaments.

The hair coloring agent may comprise one or more pigments having a $D_{50}$ particle diameter of from 5 micron to 60 micron. Particle diameter is represented by $D_{50}$, which is the median diameter by volume. $D_{50}$ is measured with a Malvern Mastersizer 2000, which is a laser diffraction particle sizer and it is measured according to ISO 13320:2009(en)

with Hydro 2000G or Hydro 2000S where the dispersant is water or ethanol. Detection range is from 0.02 micron to 2000 micron. $D_{50}$ is expressed as $x_{50}$ in ISO 13320:2009(en). Laser diffraction measures particle size distributions by measuring the angular variation in intensity of light scattered as a laser beam passes through a dispersed particulate sample analyser and the particle size is reported as a volume equivalent sphere diameter. A discussion of calculating $D_{50}$ is provided in Barber et al, Pharmaceutical Development and Technology, 3(2), 153-161 (1998).

The hair coloring agent may comprise one or more pigments having a $D_{50}$ particle diameter of from 10 micron to 40 micron. The one or more pigments may be present in the hair coloring composition in an undissolved form. The hair coloring composition may comprise from 0.01% to 25%, or from 0.1% to 20% of one or more pigments, or from 1% to 15%, or from 4% to 10% of the one or more pigments, by total weight of the composition.

The one or more pigments may be one or more colored, non-white pigments. The one or more pigments may be preferably selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments). The one or more pigments may be more preferably selected from the group consisting of are titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

The light reflecting on the metallic pigments can provide a metallic lustre effect for the overall hair style, and at the same time highlight the colored hair fibers at the regions of the hair too dark that needs more glowing effect. It can also modify the color of the light emitted through the filaments, because the pigment acts as a filter, selectively transmitting predetermined wavelengths of light. Because of this effect, the color of the surrounded hair fibers can be further highlighted.

The one or more pigments may be one or more pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, and carmine. The color exhibited by the one or more pigments can be adjusted by varying the layer thickness. Such pigments are sold, for example, under the trade names Rona®, Colorona®, Dichrona®, RonaFlair®, Ronastar®, Xirona® and Timiron® all of which are available from Merck, Darmstadt, Germany. For example, Xirona® is a brand for color travel pigments that display color shifting effects depending on the viewing angle and are based on either natural mica, silica ($SiO_2$) or calcium aluminium borosilicate flakes, coated with varying layers of titanium dioxide ($TiO_2$).

Pigments from the line KTZ® from Kobo Products, Inc., 3474 So. Clinton Ave., So. Plainfield, USA, are also useful herein, in particular the Surface Treated KTZ® Pearlescent Pigments from Kobo. Particularly useful are KTZ® FINE WHITE (mica and $TiO_2$) having a $D_{50}$ particle diameter of 5 to 25 micron and also KTZ® CELESTIAL LUSTER (mica and $TiO_2$, 10 to 60 micron) as well as KTZ® CLASSIC WHITE (mica and $TiO_2$, 10 to 60 micron). Also useful are SynCrystal Sapphire from Eckart Effect Pigments, which is a blue powder comprising platelets of synthetic fluorphlogopite coated with titanium dioxide, ferric ferrocyanide and small amounts of tin oxide. Also useful is SYNCRYSTAL Almond also from Eckart, which is a beige powder with a copper reflection color and is composed of platelets of synthetic fluorphlogopite and coated with titanium dioxide and iron oxides. Also useful is Duocrome® RV 524C from BASF, which provides a two color look via a lustrous red powder with a violet reflection powder due to its composition of mica, titanium dioxide and carmine.

The one or more pigments may be one or more organic pigments. The one or more organic pigments may be selected from the group consisting of natural pigments sepia, gamboge, bone charcoal, Cassel brown, indigo, chlorophyll and other plant pigments.

The one or more organic pigments may be one or more synthetic organic pigments. The one or more synthetic organic pigments may be selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

The one or more pigments may be selected from the group consisting of iron oxide, titanium dioxide, mica, borosilicate, and combinations thereof. The one or more pigments may comprise one or more iron oxide ($Fe_2O_3$) pigments. The one or more pigments may comprise a combination of mica and titanium dioxide.

Colored material

The hair coloring agent may comprise one or more colored materials, preferably may consist essentially of one or more colored materials. The one or more colored materials may be particulate in form. The one or more colored materials may be selected from the group consisting of colored fibers, colored beads, colored particles such as nanoparticles, colored polymers comprising covalently attached dyes, liquid crystals, particles having diffraction properties, UV absorber and photoprotective substances, pressure- or light-sensitive pigments, and combinations thereof.

The one or more colored materials may be capable of changing color via a mechanism selected from the group consisting of thermochromism, photochromism, hydrochromism, magnetochromism, electrochromism, piezochromism, chemichromism, mechano-optics. Suitable materials may include 3D Magnetic Pigments, Glow Dust, Fluorescent Pigments, Thermo Dust, Chameleon Pigments and other color changing materials from Solar Color Dust (http://solarcolordust.com/).

The hair coloring agent may comprise one or more photoprotective substances. The hair coloring composition may comprise from 0.01% to 10%, or from 0.1% to 5%, or from 0.2% to 2% of the one or more photoprotective substances, by total weight of the composition. Useful photoprotective substances are specified in EP1084696A1 from §0036 to §0053. The one or more photoprotective substances may be selected from the group consisting of 2-ethylhexyl 4-methoxy-cinnamate, methyl methoxycinnamate, 2-hydroxy-4-methoxybenzophenone-5-sulfonic acid, polyethoxylated p-aminobenzoates, di-butyl-hydroxytoluene (BHT), and mixtures thereof.

The hair coloring composition may comprise from 0.01% to 10%, or from 0.05% to 5% of one or more particulate substances, by total weight of the composition. The one or more particulate substances may be substances which are solid at room temperature (23°C) and may be in the form of particles. The one or more particulate substances may be selected from the group consisting of silica, silicates, aluminates, clay earths, mica, and insoluble salts. The one or more particulate substances may be selected from the group consisting of insoluble inorganic metal salts, metal oxides, minerals and insoluble polymer particles. The one or more particulate substances may be titanium dioxide.

The one or more particulate substances may be present in the hair coloring composition in undissolved, or stably dispersed form, and, following application to the hair and evaporation of the solvent, can deposit on the hair in solid form.

The light reflecting on the colored materials can provide a colored lustre effect for the overall hair style, and at the same time highlight the colored hair fibers at the regions of the hair too dark that needs more glowing effect. It can also modify the color of the light emitted through the filaments, because the colored materials can act as a filter, selectively transmitting predetermined wavelengths of light. Because of this effect, the color of the surrounded hair fibers can be also further highlighted.

Optional ingredients for the hair coloring composition

The hair coloring composition according to the present invention may comprise, in addition to the ingredients indicated above, further ingredients in order to further enhance the properties of the hair coloring composition, as long as these are not excluded by the claims.

Suitable further optional ingredients include, in a cosmetically acceptable carrier, but not limited to: solvents; ether of water-soluble polyhydric alcohol, thickening system, anti-freeze agent; alkalizing agents; chelants; radical scavengers; pH modifiers and buffering agents; rheology modifiers; carbonate ion sources; peroxymonocarbonate ion sources; surfactants; perfume; enzymes; dispersing agents; peroxide stabilizing agents; antioxidants; natural ingredients (such as proteins, protein compounds, and plant extracts); conditioning agents (such as silicones and cationic polymers); ceramides; preservatives; opacifiers and pearling agents (such as titanium dioxide and mica); and mixtures thereof. The further optional ingredients may be present in the hair coloring composition in an amount ranging from 0% to 20% by weight, relative to the total weight of the hair coloring composition. Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

Thickening system

The hair coloring composition may comprise a thickening system. The thickening system may comprise a deposition enhancer and a thickening polymer. The hair coloring composition may comprise from 0.5% to 10% of a thickening system by total weight of the composition. The selected thickening system can help to prevent dripping of the hair coloring composition by inhibiting capillary action, which would otherwise quickly draw the hair coloring composition down the light-transmissive filaments onto the hair fibers and onto the floor and/or clothes of the user. Nevertheless, the thickening system, particularly the combination of thickening polymer and deposition enhancer, provide a non-dripping composition that adheres well to the filaments and optionally also to the keratin fibers without generating a sticky feel. Moreover, the thickening system does not detract from the ability of the film-forming aminosilicone polymer to provide the thin, dry film.

The thickening system may comprise a deposition enhancer. The deposition enhancer may be a hydrophilic and non-ionic polymer, and wherein the deposition enhancer has a weight average molecular weight of from 700,000 Dalton to 3,000,000 Dalton. The deposition enhancer may be useful for aiding the deposition of the film-forming aminosilicone polymer as well as any hair coloring agent on to the light-transmissive filaments. In particular, the deposition enhancer may have the advantage that more film-forming aminosilicone polymer as well as any hair coloring agent stays on the light-transmissive filaments rather than dripping or sliding off the filament. Indeed, in view of the physical structure the plurality of light-transmissive filaments that are close proximity to one another, capillary action can play a role with regards to fluids on filaments. Hence, the deposition enhancer can be useful for preventing the capillary action from stripping the film-forming aminosilicone polymer as well as any hair coloring agent from the light-transmissive filaments.

The hair coloring composition may comprise from 0.01% to 10% of a deposition enhancer by total weight of the composition. The hair coloring composition may comprise from 0.05% to 4%, or from 0.075% to 3.5%, or from 0.1% to 3%, or from 0.1% to 2%, or from 0.15% to 1% of a deposition enhancer, by total weight of the composition.

The deposition enhancer may conform to the formula $H(OCH_2CH_2)_nOH$ wherein n has an average value of from 20,000 to 50,000. The deposition enhancer may conform to the formula $H(OCH_2CH_2)_nOH$ wherein n has an average value of from 40,000 to 50,000. The hair coloring composition may comprise from 0.01% to 10% of a deposition enhancer by total weight of the composition. The hair coloring composition may comprise from 0.05% to 4%, or from 0.075% to 3.5%, or from 0.1% to 3%, or from 0.1% to 2%, or from 0.15% to 1% of a deposition enhancer, by total weight of the composition, wherein the deposition enhancer conforms to the formula $H(OCH_2CH_2)_nOH$ wherein n has an average value of from 40,000 to 50,000.

Useful deposition enhancers are available from Dow under their POLYOX brand. In particular, POLYOX WSR N-60K is PEG-45M i.e. formula $H(OCH_2CH_2)_nOH$ wherein n is an integer and where n has an average value of 45,000. PEG-45M has a weight average molecular weight of 2,000,000 Dalton. Also useful is POLYOX WSR N-12K, which is PEG-23M i.e. formula $H(OCH_2CH_2)_nOH$ wherein n is an integer where n has an average value of 23,000. PEG-23M has a weight average molecular weight of 1,000,000 Dalton. Also useful is POLYOX WSR-1105, which has a weight average molecular weight of 900,000 Dalton.

The thickening system may comprise a thickening polymer. The thickening polymer may have a weight average molecular weight of at least 10,000 Dalton. The thickening polymer may be a cationic thickening polymer or is a non-ionic thickening polymer. The hair coloring composition may comprise from 0.01% to 10% of a thickening polymer by total weight of the composition. The hair coloring composition may comprise from 0.1%, or from 0.2%, or from 0.3%, or from 0.4, or from 0.5%, or from 0.6%, or from 0.7%, or from 0.8%, or from 0.9% to 5%, or to 4.5%, or to 4%, or to 3.5%, or to 3%, or to 2.5%, or to 2%, or to 1.5% of a thickening polymer by total weight of the composition. The thickening polymer may be a non-ionic thickening polymer.

The thickening polymer may be a polysaccharide. The thickening polymer may be a polysaccharide and the polysaccharide may be selected from the group consisting of hydroxyethylcellulose, hydroxypropylcellulose, starch compounds, xanthan gum, carrageenans, and mixtures thereof. The thickening polymer may be a heteropolysaccharide. The total polysaccharide content present in the hair coloring composition may be from 0.2% to 5%, or from 0.5% to 4%, by total weight of the composition. Suitable polysaccharides and heteropolysaccharides may include starches and derivatives thereof, e.g. mono- or di-esters with phosphoric acid, cellulose types and their derivatives, xanthan gums, carrageenans. Heteropolysaccharides may include xanthan gum such as Keltrol® from Kelco, and Natrosol® 250 HHR from Herkules. The viscosity-increasing agent may be a starch compound. The viscosity-increasing agent may be a hydroxypropyl starch phosphate. An example of a hydroxypropyl starch phosphate may be Structure® XL from Akzo Nobel. The thickening polymer may be a hydroxethyl cellulose. A suitable hydroxethyl cellulose is Cellosize™ HEC QP 4400 from Dow.

The thickening polymer may be a cationic thickening polymer. The thickening polymer may comprise a hydrocarbon backbone substituted with an amino-group containing sidechain. The thickening polymer may be a cationic thickening polymer comprising a quaternary amine group, alternatively a quaternary ammonium group. The thickening polymer may be a Polyquaternium-37. Polyquaternium-37 is a poly(2-methacryloxyethyltrimethylammonium chloride). Polyquaternium-37 is available from BASF via the product Salcare® SC 96 FROM BASF, which has the INCI name: Polyquaternium-37 (and) Propylene Glycol Dicaprate Dicaprylate (and) PPG-1 Trideceth-6. Polyquaternium-37. Polyquaternium-37 is also available as: Syntran PC 5320 (Interpolymer Corporation); Ultragel® 300 from Cognis GmbH; OriStar PQ37 from Orient Stars LLC; Synthalen CN from 3V Group; Synthalen CR from 3V Group; Synthalen CU from 3V Group; Cosmedia® Triple C from Cognis GmbH, which has the INCI: Polyquaternium-37, Dicaprylyl Carbonate, Lauryl Glucoside.

Anti-freeze agent

The hair coloring composition may comprise from 0.5% to 30% of a volatile alcohol, wherein the volatile alcohol may have from 1 to 8 carbon atoms and may be miscible in water. The volatile alcohol may act as an anti-freeze agent (or a first anti-freeze agent). An anti-freeze agent has the advantage that it lowers the freezing point of a composition and consequently prevents the composition from freezing, which can prevent any unwanted side effects of freezing and/or subsequent thawing.

The hair coloring composition may comprise from 0.5% to 3%, or from 0.75% to 2.5%, or from 1% to 2% of a volatile alcohol by total weight of the composition, wherein the volatile alcohol may have from 1 to 8 carbon atoms and may be miscible in water. The volatile alcohol may be a monohydric alkyl alcohol having from 1 to 4 carbon atoms. The volatile alcohol may be selected from the group consisting of alkoxylated alcohols having a maximum of 8 carbon atoms, ethanol, propanol, isopropanol, and mixtures thereof. The volatile alcohol may be selected from the group consisting of isopropanol, $CH_3CH_2OCH_2CH_2OCH_2CH_2OH$, $CH_3OCH_2CH(CH_3)OCH_2CH(CH_3)OH$, $CH_3CH(OH)CH_2OCH_2CH(CH_3)OCH_3$, $CH_3CH_2OH$, and mixtures thereof. Miscibility in water can be useful in view of the advantages of using water as a solvent. Indeed, the solvent may be water and/or the majority of the solvent may be water. Since the present first aspect is "for providing a film on keratin fibers", the volatility of the alcohol can be chosen such that solvent and other non-film-forming compounds evaporate. A suitable product for providing the volatile alcohol can be Aquasolved Super from Firmenich.

The hair coloring composition may comprise a first anti-freeze agent and a further anti-freeze agent. The further anti-freeze agent may modify the effects of the first anti-freeze agent, for example, by altering any negative effects of the first anti-freeze agent. The hair coloring composition may comprise from 0.1% to 2%, or from 0.2% to 1% of a further anti-freeze agent by total weight of the composition. The further anti-freeze agent may be a surfactant, for example a non-ionic surfactant. The further anti-freeze agent may be a non-ionic surfactant having from 20 to 50 carbon atoms, or from 22 to 25 carbon atoms. The non-ionic surfactant may be an ether of a polyethylene glycol. The non-ionic surfactant may be a $C_{5-32}$ alkyl alcohol ether of a polyethylene glycol. The non-ionic surfactant may be a $C_{10-25}$ alkyl alcohol ether of a polyethylene glycol. Said polyethylene glycol may conform to the formula $H(OCH_2CH_2)_nOH$ wherein n is an integer from 1 to 20, or from 5 to 12. The non-ionic surfactant may be an ether of triglycerin, hexaglycerin, PPG-4, PPG-6, PEG-5, PEG-6, PEG-8, PEG-12, PEG-14, PEG-18, or PEG-20. A suitable non-ionic surfactant may be Tergitol™ 15-S-9 from Dow, which has the INCI name C11-15 Pareth-9 and may be the polyethylene glycol ether of a mixture of synthetic $C_{11-15}$ fatty alcohols with an average of 9 moles of ethylene oxide. Another suitable non-ionic surfactant may be trideceth-12.

Preservative

The hair coloring composition may comprise at least one preservative and/or a mixture of preservatives. The preservative and/or mixture of preservatives may be active against gram negative bacteria, *Staphylococcus aureus* and *Candida albicans.* The hair coloring composition may comprise 2-phenoxyethanol and/or phenylmethanol. The hair coloring composition may comprise 2-phenoxyethanol. The composition may comprise from 0.01% to 5% of a preservative, or from 0.1% to 2%, or from 0.5% to 1.5% of a preservative by total weight of the composition. The preservative may be selected from the group consisting of benzyl alcohol, phenoxyethanol, and mixtures thereof. The hair coloring composition may comprise at least one preservative; and wherein the preservative may be selected from the group consisting of benzyl alcohol and phenoxyethanol; or wherein the preservative may be a mixture of benzyl alcohol and phenoxyethanol.

The hair coloring composition may be substantially free of esters of parahydroxybenzoic acid. Esters of parahydroxybenzoic acid are commonly known as parabens.

The hair coloring composition may be substantially free of isothiazolinone compounds. The hair coloring composition may be substantially free of benzoate compounds. The composition may be substantially free of 1,3-bis(hydroxymethyl)-5,5-dimethylimidazolidine-2,4-dione.

Rheology

The hair coloring composition may have a viscosity of from 30 mPa·s to 1000 mPa·s. The viscosity is measured at 23°C using a HAAKE Rotation Viscometer VT 550 with cooling/heating vessel and sensor systems according to DIN 53019 at a shear rate of 12.9 $s^{-1}$. The hair coloring composition may have a viscosity of from 100 mPa·s to 500 mPa·s, or from 150 mPa·s to 450 mPa·s, or from 200 mPa·s to 400 mPa·s, or from 250 mPa·s to 350 mPa·s. The hair coloring composition may have a viscosity of from 100 mPa·s to 200 mPa·s. The viscosity range is useful in view of helping to prevent the hair coloring composition from dripping onto clothes and/or surrounding material. Furthermore, when the viscosity is too high, the hair coloring composition cannot easily be mixed.

The hair coloring composition may have a tangent delta of less than 2 at an angular frequency of 1 Hz at 23°C and at 1% strain. Tangent delta (also known as: tan delta, tan [$\delta$], loss tangent, loss factor) is the ratio of viscous modulus (G") to elastic modulus (G') and is a quantifier of the presence and extent of elasticity in a fluid. How to calculate the tan delta is shown below:

**G' = Storage Modulus**
**G" = Loss Modulus**

**tan $\delta$ = Loss Factor**

$$\frac{G''}{G'} = \tan\delta$$

$$|G^*|(w) = G'(w) + iG''(w) = \sqrt{G'^2 + G''^2}$$

The storage modulus and tangent delta may be measured using a TA-Instruments AR2000ex rheometer. In the experiment, all the components of the hair coloring composition are mixed together, and then homogenised using an IKA KS 501 digital at 160 rpm and then loaded it onto the rheometer. The rheometer is used with the following specifications/conditions: upper geometry, 60 mm steel; lower geometry, Peltier Element; temperature, 23 °C; Pre-shear, 5 1/s, 1 min; equilibration, 2 min; normal force, off; gap: 650 $\mu$m. An amplitude sweep (strain sweep) was carried out with a strain of between 0.05% and 50%, with log data sampling (10 points/decade) and at a frequency of 1 Hz (6.283 rad/s).

The hair coloring composition may have a tangent delta of from 0.6 to 2 at an angular frequency of 1 Hz at 23°C and at 1% strain. The hair coloring composition may have a tangent delta of from 0.6 to 1.5, or from 0.6 to 1.0 at an angular frequency of 1 Hz at 23°C and at 1% strain.

Method of customizing the colored hair ornament

Another aspect relates to a method for customizing the colored hair ornament as set out herein before, the method comprising at least one or any combination of two or more of:

mechanically altering the surface of at least one of the plurality of light-transmissive filaments;
cutting at least one of the plurality of light-transmissive filaments;
applying a hair coloring agent or a hair coloring composition to the surface of at least one of the plurality of light-transmissive filaments; and
applying a heat treatment to at least one of the plurality of light-transmissive filaments,
wherein said customizing is preferably performed after attaching the hair ornament to the head of the wearer.

The method may begin with a first step, in which the colored hair ornament 100 is attached to the hair of a wearer. The attachment may be achieved in any suitable way. Preferably, the colored hair ornament 100 may comprise a fastening for releasably attaching the hair ornament to the hair or head of the wearer. The fastening may comprise a hair-band, grip, clip, pin, comb, jaws or clamp, for example.

After the hair ornament is attached to the hair, at least one of the filaments is mechanically altered, in a second step. The mechanical alteration may comprise abrading, scraping, or scratching the filament to modify its optical properties. For example, by scraping a blade of a scissors along a section of the filament, the cladding may be damaged or removed in that section. This can increase the escape of light from that section of the filament, so that the filament glows brighter there.

Next, one or more of the filaments may be cut. This can allow the hair ornament to be incorporated into a hair style in a more integrated fashion, for example by cutting the hair ornament to the same length as the wearer's hair.

In another step, a hair coloring agent, such as a pigment, or a hair coloring composition may be applied to one or more of the filaments. The hair coloring agent may be applied to the filaments at the same time as applying it to the wearer's hair. Again, this can allow the colored hair ornament to be integrated to a greater extent into the hairstyle. It can also modify the color of the light emitted through the filaments, because the pigment acts as a filter, selectively transmitting predetermined wavelengths of light.

In another, a heat treatment may be applied to the hair. This may comprise curling the hair using curling tongs. The heat treatment may be applied also to the filaments which are embedded in the hair. In this way, the filaments can be curled similarly to the hair. Care is preferably taken to ensure that the temperature is not too hot and/or is not applied for too long, so as to avoid melting the filaments.

The method may be preferably carried out by a hair dresser, stylist or colorist, in a hair salon, concurrently with cutting, styling, or coloring the wearer's hair.

Kit

A kit is provided and comprises:

(a) a hair ornament wherein the hair ornament comprises a light source and a plurality of light-transmissive filaments; wherein each light-transmissive filament has a proximal end, a distal end and a side wall, wherein the proximal end of each light-transmissive filament is arranged to receive light from the light source; and

(b) a hair coloring composition, wherein the hair coloring composition comprises a film-forming aminosilicone polymer and a hair coloring agent;

(c) optionally an applicator to coat the hair coloring composition to one or more light-transmissive filaments of the plurality of light-transmissive filaments.

The kit may preferably consist of a packaging comprising the hair ornament, the hair coloring composition as set out herein above and an applicator comprising a brush or sponge head; and wherein the hair coloring composition may comprise one or more pigments and/or one or more colored materials. The kit may comprise a multi-compartment package comprising a first compartment, a second compartment and a third compartment; wherein the first compartment may comprise the hair ornament, the second compartment may comprise the hair coloring composition as set out herein above and the third compartment may comprise an applicator.

The kit may comprise a first hair coloring composition comprising a first pigment and a second hair coloring composition comprising a second pigment; wherein the first pigment and the second pigment may exhibit different colors.

The kit may also comprise an instructional material for use of the hair ornament and how to coat the hair color composition on the surfaces of the plurality of light-transmissive filaments. The instructional material can contain instructions how to use the hair color composition with the applicator. Also, the instructional material can contain further instructions how to prepare the hair coloring composition.

EXAMPLES

The following examples further describe and demonstrate embodiments within the scope of the present invention. The examples are given solely for the purpose of illustration, and are not to be construed as limitations of the present invention since many variations thereof are possible without departing from its scope.

Hair ornaments E1-E4

**Example E1**

In a first Example E1, the light-transmissive filament was a plastic optical fiber marketed by Asahi Kasei E-Materials Corporation under the product designation DB-175. This comprises a PMMA core and a fluorinated polymer cladding.

The hair ornament of Example E1 comprised a housing having a hair barrette as a fastening means, a power supply coupled to the housing, a light source, which was a LED, electrically coupled to the power supply and a plurality of about 30 light-transmissive filaments positioned to receive light from the light source and having fiber ends protruding out and away from the housing.

The housing comprised two pieces, a case with hollow compartments, and a sliding lid, which when closed concealed the compartments, or when open allowed access to the compartments. The power supply comprised two batteries to power the light source. The filaments were held in a bundle and a lens focused the light from the light source onto the end of the bundle. The filaments were held in the bundle by gluing them together at their proximal ends. They could also be crimped together at their proximal ends with a crimp sleeve or collar.

**Example E2**

In the Example E2, the starting light-transmissive filament for this example was a plastic optical fiber marketed by Asahi Kasei E-Materials Corporation under the product designation DB-175. This comprises a PMMA core and a fluorinated polymer cladding.

The distal ends of a bundle of about 30 fibers were cut at a 90-degree angle and coated with a reflective coating. A thin silver film was coated onto the distal end surfaces of the plurality of the light-transmissive filaments using a chemical technique with Tollen's reagent.

The preparation of Tollen's reagents started with adding 2% ammonia solution drop-by-drop into 2 mL of 0.1 M silver nitrate solution. After the brown precipitate was totally dissolved, 1.4 mL of 0.8 M KOH was added to form a black precipitate in the solution. Subsequently, ammonia solution was added drop wise into the solution. Once the black precipitate was dissolved, Tollen's reagent was acquired.

Before silver mirror coating, the distal end surfaces of the plurality of the light-transmissive filaments were dipped into a 0.2% tin (II) chloride dehydrate solution to sensitize the coating area. After rinsing thoroughly with deionized

water and blow-drying with dinitrogen, the coating area was dipped into Tollen's reagent. Subsequently, 0.4 mL of 0.25 M glucose was rapidly added; the distal end surfaces of the plurality of the light-transmissive filaments were then kept in the reagent for a period of 5 min to form the silver mirror. Afterwards, the plurality of the light-transmissive filaments was removed from the reagent and rinsed with deionized water and blow-dried with dinitrogen.

The hair ornament of Example E2 comprised a housing having a hair barrette as a fastening means, a power supply coupled to the housing, a light source, which was a LED, electrically coupled to the power supply and a plurality of the above about 30 light-transmissive filaments positioned to receive light from the light source and having fiber ends protruding out and away from the housing.

The housing comprised two pieces, a case with hollow compartments, and a sliding lid, which when closed concealed the compartments, or when open allowed access to the compartments. The power supply comprised two batteries to power the light source. The filaments were held in a bundle and a lens focused the light from the light source onto the end of the bundle. The filaments were held in the bundle by gluing them together at their proximal ends. They could also be crimped together at their proximal ends with a crimp sleeve or collar.

## Example E3

In the Example E3, the starting light-transmissive filament for this example was a plastic optical fiber marketed by Asahi Kasei E-Materials Corporation under the product designation DB-175. This comprises a PMMA core and a fluorinated polymer cladding.

The distal ends of a bundle of about 30 fibers were cut at a 45-degree angle to maximize brightness of light points.

The hair ornament of Example E3 comprised a housing having a hair barrette as a fastening means, a power supply coupled to the housing, a light source, which was a LED, electrically coupled to the power supply and a plurality of the above about 30 light-transmissive filaments positioned to receive light from the light source and having fiber ends protruding out and away from the housing.

The housing comprised two pieces, a case with hollow compartments, and a sliding lid, which when closed concealed the compartments, or when open allowed access to the compartments. The power supply comprised two batteries to power the light source. The filaments were held in a bundle and a lens focused the light from the light source onto the end of the bundle. The filaments were held in the bundle by gluing them together at their proximal ends. They could also be crimped together at their proximal ends with a crimp sleeve or collar.

## Example E4

In the Example E4, the light-transmissive filaments were prepared by laser ablation. The starting light-transmissive filament for this example was a plastic optical fiber marketed by Asahi Kasei E-Materials Corporation under the product designation DB-175. This comprises a PMMA core and a fluorinated polymer cladding.

A carbon dioxide ($CO_2$) laser with wavelength 10 $\mu$m and 30W power was used for laser ablation. The laser is designed to cut plastic and textiles but has an engraving mode at which it emits approximately 25% of the maximum output power. This engraving mode was used to ablate the cladding selectively.

A bundle of about 30 fibers were engraved together. Each fiber had a diameter of 175 $\mu$m $\pm$15 $\mu$m. The fibers were laid out side by side. The fibers were engraved intermittently along their length by pulsing the laser to create separate exposed areas. The fibers were held fixed. The laser was scanned across the bundle, to engrave all of the side-by-side fibers, and scanned longitudinally along the length of the fibers to engrave each fiber intermittently over its length. The exposed areas where the cladding was ablated by the laser were arranged more densely toward the distal end of the fibers and less densely towards the proximal end. The density gradient was approximately linear over the length of each fiber. The size of each exposed area was substantially constant over the length of each fiber, while the spacing between exposed areas reduced (approximately linearly) from the proximal end to the distal end. The laser beam was oriented substantially perpendicular to the longitudinal axis of the fibers. The cladding was removed on the side of the fibers facing the laser source. It was also removed on the side facing away from the laser source, because the core acts like a cylindrical lens, focusing the laser beam.

The hair ornament of Example E4 comprised a housing having a hair barrette as a fastening means, a power supply coupled to the housing, a light source, which was a LED, electrically coupled to the power supply and a plurality of the above about 30 light-transmissive filaments positioned to receive light from the light source and having fiber ends protruding out and away from the housing.

The housing comprised two pieces, a case with hollow compartments, and a sliding lid, which when closed concealed the compartments, or when open allowed access to the compartments. The power supply comprised two batteries to power the light source. The filaments were held in a bundle and a lens focused the light from the light source onto the end of the bundle. The filaments were held in the bundle by gluing them together at their proximal ends. They could also be crimped together at their proximal ends with a crimp sleeve or collar.

Hair coloring compositions:

| Ingredient / Example | 1 | 2 | 3 | 4 | 5 | 6 | 7 | 8 | 9 | 10 | 11 | 12 |
|---|---|---|---|---|---|---|---|---|---|---|---|---|
| BELSIL ADM 8301 E [1] | 10 | 8 | 12 | 15 | 14 | 18 | 15 | 14 | 12 | 15 | 15 | 15 |
| Salcare® SC 96 [2] | 0.6 | 0.75 | 0.75 | 0.75 | - | 0.75 | 0.75 | - | 0.75 | 0.6 | 0.5 | 0.75 |
| 2-hydroxyethyl cellulose [3] | - | - | - | - | 0.2 | - | - | - | - | - | - | - |
| Xanthan gum [4] | - | - | - | - | - | - | - | 0.3 | - | - | - | - |
| PEG-45M [5] | 0.2 | 0.3 | - | - | 0.1 | 0.2 | 0.2 | - | - | 0.2 | 0.2 | 0.2 |
| PEG-23M [6] | - | - | 0.2 | - | - | - | - | - | 0.2 | - | - | - |
| PEG-90M [7] | - | - | - | 0.1 | - | - | - | - | - | - | - | - |
| PEG-7M [8] | - | - | - | - | 0.3 | - | - | - | - | - | - | - |
| PEG-180M [9] | - | - | - | - | - | - | - | 0.1 | - | - | - | - |
| Tergitol [$] | - | 0.5 | - | 0.5 | - | - | 0.5 | - | 0.5 | 0.5 | 0.5 | 0.5 |

| | | | | | | | | | | | | |
|---|---|---|---|---|---|---|---|---|---|---|---|---|
| Pigment § | 5.0 | 5.0 | 5.0 | - | 5.0 | 5.0 | 5.0 | 5.0 | 5.0 | 5.0 | 5.0 | 5.0 |
| Aquasolved # | - | - | - | 2.5 | 2.5 | - | - | 2.3 | - | - | - | - |
| Ethanol | 1.0 | 1.5 | 2.0 | - | - | 1.0 | 1.5 | 2.0 | - | 1.0 | 1.0 | 1.0 |
| Preservative* | 1.0 | 2.0 | 1.5 | 0.5 | 1.0 | - | - | - | 1.0 | 1.0 | 1.0 | 1.0 |
| Perfume | - | 0.1 | 0.07 | 0.1 | - | - | - | 0.2 | 0.2 | 0.1 | 0.1 | 0.1 |
| Deionised water | QSP | QSP | QSP | QSP | QSP | QSP | QSP | QSP | QSP | QSP | QSP | QSP |

KEY:

[1] = from Wacker, aqueous mixture of poly[3-((2-aminoethyl)amino)propyl]methyl(dimethyl)siloxane (hydroxyterminated), octamethylcyclotetrasiloxane, MQ resin, ethylene glycol monohexyl ether and diethyleneglycol monobutylether;

[2] = Salcare® SC 96 from BASF, which has the INCI name: Polyquaternium-37 (and) Propylene Glycol Dicaprate Dicaprylate (and) PPG-1 Trideceth-6;

[3] = Cellosize™ HEC QP 4400 from Dow;

[4] = Keltrol® from Kelco or Natrosol® 250 HHR from Herkules;

[5] = POLYOX WSR N-60K from Dow has formula $H(OCH_2CH_2)_nOH$ wherein n is an integer and where n has an average value of 45,000 and the weight average molecular weight is 2,000,000 Dalton; [6] = POLYOX WSR N-12K from Dow has the formula $H(OCH_2CH_2)_nOH$ wherein n is an integer with an average value of 23,000 and the weight average molecular weight is 1,000,000 Dalton;

[7] = POLYOX WSR-301 from Dow has the formula $H(OCH_2CH_2)_nOH$ wherein n is an integer with an average value of 90,000 and the weight average molecular weight is 4,000,000 Dalton;

[8] = POLYOX N-750 from Dow has the formula $H(OCH_2CH_2)_nOH$ wherein n is an integer with an average value of 7,000 and the weight average molecular weight is 300,000 Dalton;

[9] = POLYOX WSR-308 from Dow, as formula $H(OCH_2CH_2)_nOH$ wherein n is an integer and where n has an average value of 180,000 and has a weight average molecular weight of 8,000,000 Dalton;

$ = C11-15 Pareth-9 and is the polyethylene glycol ether of a mixture of synthetic C11-15 fatty alcohols with an average of 9 moles of ethylene oxide.

* = 2-phenoxyethanol and/or phenylmethanol;

# = from Firmenich;

§ = pigment selected from: mica and/or iron oxides; Colorona Bronze Fine; SynCrystal Almond; Xirona Le Rouge; DUOCROME RV 524C; SynCrystal Jade; Colorona Precious Gold; Ronastar Red; Syncrystal Sapphire; Impact Silver Rutile; KTZ Misterioso Pewter; and combinations thereof.

| Ingredient / Example | 1 | 2 | 3 | 4 | 5 | 6 | 7 | 8 | 9 | 10 | 11 | 12 |
|---|---|---|---|---|---|---|---|---|---|---|---|---|
| Silsoft® CLX-E | 10 | 8 | 12 | 15 | 14 | 18 | 15 | 14 | 12 | 15 | 15 | 15 |
| Salcare® SC 96 [2] | 0.6 | 0.75 | 0.75 | 0.75 | - | - | 0.75 | - | 0.75 | 0.6 | 0.5 | 0.75 |
| 2-hydroxyethyl cellulose [3] | - | - | - | - | 0.2 | - | - | - | - | - | - | - |
| Xanthan gum [4] | - | - | - | - | - | - | - | 0.3 | - | - | - | - |
| PEG-45M [5] | 0.2 | 0.3 | - | - | 0.1 | - | 0.2 | - | - | 0.2 | 0.2 | 0.2 |
| PEG-23M [6] | - | - | 0.2 | - | - | - | - | - | 0.2 | - | - | - |
| PEG-90M [7] | - | - | - | 0.1 | - | - | - | - | - | - | - | - |
| PEG-7M [8] | - | - | - | - | 0.3 | - | - | - | - | - | - | - |
| PEG-180M [9] | - | - | - | - | - | - | - | 0.1 | - | - | - | - |
| Tergitol [$] | - | 0.5 | - | 0.5 | - | - | 0.5 | - | 0.5 | 0.5 | 0.5 | 0.5 |
| Pigment [§] | 5.0 | 5.0 | 5.0 | - | 5.0 | 5.0 | 5.0 | 5.0 | 5.0 | 5.0 | 5.0 | 5.0 |

| | | | | | | | | | | | | |
|---|---|---|---|---|---|---|---|---|---|---|---|---|
| Aquasolved # | - | - | - | 2.5 | 2.5 | - | - | 2.3 | - | - | - | - |
| Ethanol | 1.0 | 1.5 | 2.0 | - | - | 1.0 | 1.5 | 2.0 | - | 1.0 | 1.0 | 1.0 |
| Preservative* | 1.0 | 2.0 | 1.5 | 0.5 | 1.0 | - | - | - | 1.0 | 1.0 | 1.0 | 1.0 |
| Perfume | - | 0.1 | 0.07 | 0.1 | - | - | - | 0.2 | 0.2 | 0.1 | 0.1 | 0.1 |
| Deionised water | QSP | QSP | QSP | QSP | QSP | QSP | QSP | QSP | QSP | QSP | QSP | QSP |

KEY:

1 = from Momentive Performance Materials, Albany, New York, (US). Silsoft® CLX-E has as the INCI name "dipropylene glycol and polysilicone-19". Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane;

2 = Salcare® SC 96 from BASF, which has the INCI name: Polyquaternium-37 (and) Propylene Glycol Dicaprate Dicaprylate (and) PPG-1 Trideceth-6;

3 = Cellosize™ HEC QP 4400 from Dow;

4 = Keltrol® from Kelco or Natrosol® 250 HHR from Herkules;

5 = POLYOX WSR N-60K from Dow has formula $H(OCH_2CH_2)_nOH$ wherein n is an integer and where n has an average value of 45,000 and the weight average molecular weight is 2,000,000 Dalton; 6 = POLYOX WSR N-12K from Dow has the formula $H(OCH_2CH_2)_nOH$ wherein n is an integer with an average value of 23,000 and the weight average molecular weight is 1,000,000 Dalton;

7 = POLYOX WSR-301 from Dow has the formula $H(OCH_2CH_2)_nOH$ wherein n is an integer with an average value of 90,000 and the weight average molecular weight is 4,000,000 Dalton;

8 = POLYOX N-750 from Dow has the formula $H(OCH_2CH_2)_nOH$ wherein n is an integer with an average value of 7,000 and the weight average molecular weight is 300,000 Dalton;

9 = POLYOX WSR-308 from Dow, as formula $H(OCH_2CH_2)_nOH$ wherein n is an integer and where n has an average value of 180,000 and has a weight average molecular weight of 8,000,000 Dalton;

$ = C11-15 Pareth-9 and is the polyethylene glycol ether of a mixture of synthetic C11-15 fatty alcohols with an average of 9 moles of ethylene oxide.

* = 2-phenoxyethanol and/or phenylmethanol;

# = from Firmenich;

§ = pigment selected from: mica and/or iron oxides; Colorona Bronze Fine; SynCrystal Almond; Xirona Le Rouge; DUOCROME RV 524C; SynCrystal Jade; Colorona Precious Gold; Ronastar Red; Syncrystal Sapphire; Impact Silver Rutile; KTZ Misterioso Pewter; copper pigment from Colorona®; and combinations thereof.

The following hair coloring compositions H1 and H2 were prepared (all amounts are in wt%). A hair coloring composition H1 comprised a MQ resin.

| | Water | Pigment§ | Z | EtOH | Thickening polymer* | Deposition enhancer# | Phenoxy-ethanol | Anti-freeze agent$ | Total |
|---|---|---|---|---|---|---|---|---|---|
| **H1** | Qsp | 5 | 15 | 2 | 0.75 | 0.2 | 1 | 0.5 | 100 |
| KEY: Z = Wacker® HC303 (commercially available); EtOH = ethanol; * = Salcare® SC96 from BASF; # = PEG-45M (Polyox WSR N-60K) from Dow; § = copper pigment from Colorona®; and $ = $C_{11-15}$ Pareth-9 and is the polyethylene glycol ether of a mixture of synthetic C11-15 fatty alcohols with an average of 9 moles of ethylene oxide. | | | | | | | | | |

A composition H2 comprised as a film-forming polymer Silsoft® CLX-E.

| Composition | Water | Pigment§ | Z (% wt.) | Total |
|---|---|---|---|---|
| H3 | QSP | 5 | 17% (2.55% active) | 100 |
| KEY: Z = from Momentive Performance Materials, Albany, New York, (US). Silsoft® CLX-E has as the INCI name "dipropylene glycol and polysilicone-19". Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane; § = copper pigment from Colorona®. | | | | |

Preparation of the colored hair ornament

1.0 g of the respective hair coloring composition H1-H2 was applied on the plurality of light-transmissive filaments of the above Examples E1-E4.

The respective hair coloring composition H1-H2 was distributed onto the light-transmissive filaments with the help of a brush or a sponge head. The light-transmissive filaments were flipped. The respective hair coloring composition H1-H2 was further distributed until all light-transmissive filaments were completely and evenly colored. Each treated light-transmissive filament was blow dried manually with a conventional blow dryer for 2 minutes.

The coating of the light-transmissive filaments was performed when the hair ornament was positioned onto the consumer's hair with the help of the hair barrette. The coating of the light-transmissive filaments could be also performed before positioning the hair ornament onto the consumer's hair.

The best lustre and metallic reflective effect was obtained with the combination of Examples E4 with H1 or H2. Each colored hair ornament led therefore to a sophisticated hair decoration which was able to highlight the hair style but also the color of the natural hair fibers and/or the color of the dyed hair fibers. The color of the light emitted through the filaments was modified because the metallic pigment acted as a filter, selectively transmitting predetermined wavelengths of light. Because of this effect, the color of the surrounded hair fibers was further highlighted.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A colored hair ornament (100) comprising:

   a light source (110); and
   a plurality of light-transmissive filaments (10, 15, 130);
   wherein each light-transmissive filament has a proximal end (40, 132), a distal end (50, 134) and a side wall,
   wherein the proximal end of each light-transmissive filament is arranged to receive light from the light source;
   wherein one or more light-transmissive filaments of the plurality of light-transmissive filaments are coated with a hair coloring composition (80);
   wherein the hair coloring composition comprises a film-forming aminosilicone polymer (70) and a hair coloring agent (75).

2. The colored hair ornament of claim 1, wherein a reflective coating is applied to the distal end of each light-transmissive filament protruding from the light source.

3. The colored hair ornament of claim 1, wherein the distal end protruding out and away from the light source is cut at an angle to maximize observed brightness of light emitted from the light source, wherein the angle ranges from 15 to 75 degrees.

4. The colored hair ornament of claim 1, wherein each light-transmissive filament is configured to emit through the side wall at least some of the light received at the proximal end,
   the light-transmissive filament comprising a first segment of unit length, located at a first distance from the proximal end, the first segment being configured to emit through the side wall a first proportion of the light arriving at the first segment from the proximal end;
   the light-transmissive filament further comprising a second segment of unit length, located at a second distance from the proximal end, the second segment being configured to emit through the side wall a second proportion of the light arriving at the second segment from the proximal end;
   wherein first distance is preferably greater than the second distance; and
   wherein an optical property of the light-transmissive filament varies over a length of the filament such that the first proportion is greater than the second proportion.

5. The colored hair ornament of any preceding claim, wherein the light-transmissive filament comprises a core and a cladding, preferably wherein the core is made of PMMA.

6. The colored hair ornament of claim 5, wherein the light-transmissive filament comprises a cladding made of a fluorinated polymer.

7. The colored hair ornament of any preceding claim, wherein the light-transmissive filament comprises a layer including at least one of: keratin; and melanin.

8. The colored hair ornament of any preceding claim, wherein the film-forming aminosilicone polymer of the hair coloring composition comprises:

   (a) an aminosilicone polymer comprising amino sidechains, and wherein the aminosilicone polymer has a weight average molecular weight of from 10,000 Dalton to 60,000 Dalton; and
   (b) a silicone resin, preferably a MQ resin.

9. The colored hair ornament according to any of the claims 1 to 7, wherein the film-forming aminosilicone polymer is the product of the reaction of:

   a1) a siloxane comprising at least two oxirane or oxetane groups having the formula:

$$M_f M^E_h M^{PE}_i M^H_j D_k D^E_l D^{PE}_m D^H_n T_o T^E_p T^{PE}_q T^H_r Q_s$$

   With

   $M = R^9R^{10}R^{11}SiO_{1/2}$;

$M^H = R^{12}R^{13}HSiO_{1/2}$;
$M^{PE} = R^{12}R^{13}(-CH_2CH(R^{14})(R^{15})_tO(R^{16})_u(C_2H_4O)_v(C_3H_6O)_w(C_4H_8O)_xR^{17})SiO_{1/2}$;
$M^E = R^{12}R^{13}(R^E)SiO_{1/2}$;
D= $R^{18}R^{19}SiO_{2/2}$; and
$D^H = R^{20}HSiO_{2/2}$;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17})SiO_{3/2}$;
$T^E = R^ESiO_{3/2}$; and
$Q = SiO_{4/2}$;
Where
$R^9$, $R^{10}$, $R^{11}$, $R^{12}$, $R^{13}$, $R^{18}$, $R^{19}$, $R^{20}$, and $R^{21}$ are each independently selected from the group of monovalent hydrocarbon radicals having from 1 to 60 carbon atoms;
$R^{14}$ is H or a 1 to 6 carbon atom alkyl group;
$R^{15}$ is a divalent alkyl radical of 1 to 6 carbons;
$R^{16}$ is selected from the group of divalent radicals consisting of $-C_2H_4O-$, $-C_3H_6O-$, and $-C_4H_8O-$;
$R^{17}$ is selected from the group consisting of H, monofunctional hydrocarbon radicals of 1 to 6 carbons, and acetyl;
$R^E$ is independently a monovalent hydrocarbon radical containing one or more oxirane or oxetane moieties having from 1 to 60 carbon atoms;
the subscript f is 0 or positive subject to the limitation that when the subscript f is 0, h must be positive;
the subscript h is 0 or positive subject to the limitations that when h is 0, the subscript f is positive, and that the sum of the subscripts h, 1 and p is positive;
the subscript k is zero or positive and has a value ranging from 0 to 1000;
the subscript 1 is 0 or positive and has a value ranging from 0 to 400 subject to the limitation that the sum of the subscripts h, 1 and p is positive;
the subscript o is 0 or positive and has a value ranging from 0 to 50;
the subscript p is 0 or positive and has a value ranging from 0 to 30 subject to the limitation that the sum of the subscripts h, 1 and p is positive;
the subscript s is 0 or positive and has a value ranging from 0 to 20;
the subscript i is 0 or positive and has a value ranging from 0 to 20;
the subscript m is 0 or positive and has a value ranging from 0 to 200;
the subscript q is 0 or positive and has a value ranging from 0 to 30;
the subscript j is 0 or positive and has a value ranging from 0 to 2;
the subscript n is 0 or positive and has a value ranging from 0 to 20;
the subscript r is 0 or positive and has a value ranging from 0 to 30;
the subscript t is 0 or 1;
the subscript u is 0 or 1;
the subscript v is 0 or positive and has a value ranging from 0 to 100 subject to the limitation that $(v+w+x) > 0$;
the subscript w is 0 or positive and has a value ranging from 0 to 100 subject to the limitation that $(v+w+x) > 0$;
the subscript x is 0 or positive and has a value ranging from 0 to 100 subject to the limitation that $(v + w + x) > 0$; and

a2) an aminosilane having the formula:

$$N(H)(R^1)R^2Si(OR^3)_{3-a-b-c}(OR^4)_a(R^5Si(OR^6)_d(R^7)_e)_bR^8_c$$

with

$R^1$ is chosen from the group consisting of H or a monovalent hydrocarbon radical containing 1 to 20 carbon atoms;
$R^2$ is selected from a group consisting of a divalent linear or branched hydrocarbon radical consisting of 1 to 60 carbons;
$R^4$ is a hydrocarbon radical that contains 3 to 200 carbon atoms;
$R^5$ is selected from a group consisting of oxygen or a divalent linear or branched hydrocarbon radical consisting of 1 to 60 carbons;

$R^3$, $R^6$, $R^7$, and $R^8$ and are each independently selected from the group of monovalent linear or branched hydrocarbon radicals having from 1 to 200 carbon atoms;
the subscript b is 0 or a positive number and has a value ranging from 0 to 3;
the subscript a is a positive number less than 3;
the subscripts b and c are 0 or positive and have a value ranging from 0 to 3 subject to the limitation that $(a+b+c) \leq 3$;
the subscripts d and e are 0 or positive and have a value ranging from 0 to 3 subject to the limitation that $(d+e) = 3$.

**10.** The colored hair ornament of claim 9, wherein:
the siloxane is an epoxy encapped polysiloxane with the average structure:

$$CH_2(O)CHCH_2O(CH_2)_\lambda Si(CH_3)_2O[Si(CH_3)_2O]_k Si(CH_3)_2CH_2CH_2CH_2OCH_2CH(O)CH_2$$

with the subscript k ranges from 5 to 250, from 5 to 150, preferably from 50 to 100, and the subscript $\lambda$ is 2 or 3; and the aminosilane has the formula $H_2NR^2Si(OR^3)_2(OR^4)$ with
$R^2$ has from 2 to 5 carbon atoms;
$R^3$ has from 3 to 5 carbon atoms; and
$R^4$ has from 3 to 5 carbon atoms.

**11.** The colored hair ornament of claim 10, wherein the film-forming aminosilicone polymer is the product of the reaction of the siloxane (a1), the aminosilane (a2), and an encapped polyether with the average structure $CH_2(O)CHCH_2O(CH_2CH_2O)_\mu CH_2CH(O)CH_2$ with the subscript $\beta$ ranges from 10 to 20, or an encapped polyether with the average structure $CH_2(O)CHCH_2O(CH_2CH_2O)_\mu(CH_2CHCH_3O)_\nu CH_2CH(O)CH_2$ with the subscript $\mu$ ranges from 1 to 20 and with the subscript v ranges from 1 to 20.

**12.** The colored hair ornament of any preceding claim, wherein the hair coloring agent comprises one or more pigments, preferably wherein the one or more pigments have a $D_{50}$ particle diameter of from 5 micron to 60 micron, more preferably wherein the one or more pigments are metallic pigments.

**13.** The colored hair ornament of any preceding claim, wherein the film-forming aminosilicone polymer of the hair coloring composition comprises:
(c) a thickening system comprising:

a. a deposition enhancer, wherein the deposition enhancer is a hydrophilic and non-ionic polymer, and wherein the deposition enhancer has a weight average molecular weight of from 700,000 Dalton to 3,000,000 Dalton;
b. a thickening polymer, wherein the thickening polymer has a weight average molecular weight of at least 10,000 Dalton, and wherein the thickening polymer is a cationic thickening polymer or is a non-ionic thickening polymer.

**14.** A method of customizing a colored hair ornament (100) of any one of claims 1 to 13, comprising at least one or any combination of two or more of:

mechanically altering the surface of at least one of the plurality of light-transmissive filaments;
cutting at least one of the plurality of light-transmissive filaments;
applying a hair coloring agent or a hair coloring composition to the surface of at least one of the plurality of light-transmissive filaments; and
applying a heat treatment to at least one of the plurality of light-transmissive filaments,
wherein said customizing is preferably performed after attaching the hair ornament to the head of the wearer.

**15.** A kit comprising:

(a) a hair ornament (100) wherein the hair ornament comprises a light source (110) and a plurality of light-transmissive filaments (10, 15, 130); wherein each light-transmissive filament has a proximal end (40, 132), a distal end (50, 134) and a side wall, wherein the proximal end of each light-transmissive filament is arranged to receive light from the light source; and
(b) a hair coloring composition (80), wherein the hair coloring composition comprises a film-forming aminosilicone polymer (70) and a hair coloring agent (75);

(c) optionally an applicator to coat the hair coloring composition to one or more light-transmissive filaments of the plurality of light-transmissive filaments.

## Patentansprüche

1. Farbiger Haarschmuck (100), umfassend:

   eine Lichtquelle (110); und
   eine Vielzahl von lichtdurchlässigen Filamenten (10, 15, 130);
   wobei jedes lichtdurchlässige Filament ein proximales Ende (40, 132), ein distales Ende (50, 134) und eine Seitenwand hat, wobei das proximale Ende jedes lichtdurchlässigen Filaments so angeordnet ist, dass es Licht von der Lichtquelle empfängt;
   wobei ein oder mehrere lichtdurchlässige Filamente der Vielzahl von lichtdurchlässigen Filamenten mit einer Haarfärbezusammensetzung (80) beschichtet sind;
   wobei die Haarfärbezusammensetzung ein filmbildendes Aminosilikonpolymer (70) und ein Haarfärbemittel (75) umfasst.

2. Farbiger Haarschmuck nach Anspruch 1, wobei eine reflektierende Beschichtung auf das distale Ende jedes lichtdurchlässigen Filaments aufgebracht wird, das von der Lichtquelle hervorsteht.

3. Farbiger Haarschmuck nach Anspruch 1, wobei das distale Ende, das von der Lichtquelle hervor- und wegsteht, in einem Winkel abgeschnitten wird, um die beobachtete Helligkeit des Lichts zu maximieren, das von der Lichtquelle abgegeben wird, wobei der Winkel von 15 bis 75 Grad reicht.

4. Farbiger Haarschmuck nach Anspruch 1, wobei jedes lichtdurchlässige Filament so konfiguriert ist, dass es durch die Seitenwand zumindest etwas des Lichts abgibt, das am proximalen Ende empfangen wird,
   wobei das lichtdurchlässige Filament ein erstes Segment der Einheitslänge umfasst, das sich in einem ersten Abstand vom proximalen Ende befindet, wobei das erste Segment so konfiguriert ist, dass es durch die Seitenwand einen ersten Anteil des Lichts abgibt, das am ersten Segment vom proximalen Ende ankommt;
   wobei das lichtdurchlässige Filament ferner ein zweites Segment der Einheitslänge umfasst, das sich in einem zweiten Abstand vom proximalen Ende befindet, wobei das zweite Segment so konfiguriert ist, dass es durch die Seitenwand einen zweiten Anteil des Lichts abgibt, das am zweiten Segment vom proximalen Ende ankommt;
   wobei der erste Abstand bevorzugt größer ist als der zweite Abstand; und
   wobei eine optische Eigenschaft des lichtdurchlässigen Filaments sich über eine Länge des Filaments unterscheidet, sodass der erste Anteil größer ist als der zweite Anteil.

5. Farbiger Haarschmuck nach einem der vorhergehenden Ansprüche, wobei das lichtdurchlässige Filament einen Kern und einen Mantel umfasst, wobei der Kern bevorzugt aus PMMA hergestellt ist.

6. Farbiger Haarschmuck nach Anspruch 5, wobei das lichtdurchlässige Filament einen Mantel umfasst, der aus einem fluorierten Polymer hergestellt ist.

7. Farbiger Haarschmuck nach einem der vorhergehenden Ansprüche, wobei das lichtdurchlässige Filament eine Schicht umfasst, die mindestens eines der Folgenden beinhaltet: Keratin und Melanin.

8. Farbiger Haarschmuck nach einem der vorhergehenden Ansprüche, wobei das filmbildende Aminosilikonpolymer der Haarfärbezusammensetzung Folgendes umfasst:

   (a) ein Aminosilikonpolymer, das Aminoseitenketten umfasst, und wobei das Aminosilikonpolymer ein gewichtsgemitteltes Molekulargewicht von 10.000 Dalton bis 60.000 Dalton aufweist; und
   (b) ein Silikonharz, bevorzugt ein MQ-Harz.

9. Farbiger Haarschmuck nach einem der Ansprüche 1 bis 7, wobei das filmbildende Aminosilikonpolymer das Produkt der Reaktion von Folgendem ist:

   a1) einem Siloxan, das mindestens zwei Oxiran- oder Oxetan-Gruppen mit der folgenden Formel umfasst:

$$M_f M^E_h M^{PE}_i M^H_j D_k D^E_l D^{PE}_m D^H_n T_o T^E_p T^{PE}_q T^H_r Q_s,$$

wobei

$M = R^9R^{10}R^{11}SiO_{1/2}$;
$M^H = R^{12}R^{13}HSiO_{1/2}$;
$M^{PE} = R^{12}R^{13} (-CH_2CH(R^{14})(R^{15})_tO(R^{16})_u(C_2H_4O)_v(C_3H_6O)_w(C_4H_8O)_xR^{17})SiO_{1/2}$;
$M^E = R^{12}R^{13}(R^E)SiO_{1/2}$;
$D = R^{18}R^{19}SiO_{2/2}$; und
$D^H = R^{20}HSiO_{2/2}$;
$D^{PE} = R^{20}(-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17})SiO_{3/2}$;
$T^E = R^ESiO_{3/2}$; und
$Q = SiO_{4/2}$;
Wobei
$R^9$, $R^{10}$, $R^{11}$, $R^{12}$, $R^{13}$, $R^{18}$, $R^{19}$, $R^{20}$ und $R^{21}$ jeweils unabhängig aus der Gruppe einwertiger Kohlenwasserstoffreste ausgewählt wird, die von 1 bis 60 Kohlenstoffatome aufweisen;
$R^{14}$ H oder eine Alkylgruppe mit 1 bis 6 Kohlenstoffatomen ist;
$R^{15}$ ein zweiwertiger Alkylrest von 1 bis 6 Kohlenstoffen ist;
$R^{16}$ aus der Gruppe von zweiwertigen Resten ausgewählt wird, die aus $-C_2H_4O-$, $-C_3H_6O-$ und $-C_4H_8O-$ bestehen;
$R^{17}$ aus der Gruppe ausgewählt wird, die aus H, monofunktionellen Kohlenwasserstoffresten von 1 bis 6 Kohlenwasserstoffen und Acetyl besteht;
$R^E$ unabhängig ein einwertiger Kohlenwasserstoffrest ist, der ein oder mehrere Oxiran- oder Oxetan-Einheiten enthält, die von 1 bis 60 Kohlenstoffatome aufweisen;
das tiefgestellte f 0 oder positiv ist, mit der Einschränkung, dass, wenn das tiefgestellte f gleich 0 ist, h positiv sein muss;
das tiefgestellte h 0 oder positiv ist, mit der Einschränkung, dass, wenn h gleich 0 ist, das untergestellte f positiv ist und die Summe der tiefgestellten h, l und p positiv ist;
das tiefgestellte k null oder positiv ist und einen Wert hat, der von 0 bis 1000 reicht;
das tiefgestellte l 0 oder positiv ist und einen Wert hat, der von 0 bis 400 reicht, mit der Einschränkung, dass die Summe der tiefgestellten h, l und p positiv ist;
das tiefgestellte o 0 oder positiv ist und einen Wert hat, der von 0 bis 50 reicht;
das tiefgestellte p 0 oder positiv ist und einen Wert hat, der von 0 bis 30 reicht, mit der Einschränkung, dass die Summe der tiefgestellten h, l und p positiv ist;
das tiefgestellte s 0 oder positiv ist und einen Wert hat, der von 0 bis 20 reicht;
das tiefgestellte i 0 oder positiv ist und einen Wert hat, der von 0 bis 20 reicht;
das tiefgestellte m 0 oder positiv ist und einen Wert hat, der von 0 bis 200 reicht;
das tiefgestellte q 0 oder positiv ist und einen Wert hat, der von 0 bis 30 reicht;
das tiefgestellte j 0 oder positiv ist und einen Wert hat, der von 0 bis 2 reicht;
das tiefgestellte n 0 oder positiv ist und einen Wert hat, der von 0 bis 20 reicht;
das tiefgestellte r 0 oder positiv ist und einen Wert hat, der von 0 bis 30 reicht;
das tiefgestellte t 0 oder 1 ist;
das tiefgestellte u 0 oder 1 ist;
das tiefgestellte v 0 oder positiv ist und einen Wert hat, der von 0 bis 100 reicht, mit der Einschränkung, dass $(v+w+x) > 0$;
das tiefgestellte w 0 oder positiv ist und einen Wert hat, der von 0 bis 100 reicht, mit der Einschränkung, dass $(v+w+x) > 0$;
das tiefgestellte x 0 oder positiv ist und einen Wert hat, der von 0 bis 100 reicht, mit der Einschränkung, dass $(v + w + x) > 0$; und

a2) ein Aminosilan mit der Formel:

$$N(H)(R^1)R^2Si(OR^3)_{3-a-b-c}(OR^4)_a(R^5Si(OR^6)_d(R^7)_e)_bR^8_c,$$

wobei

$R^1$ aus der Gruppe ausgewählt wird, die aus H oder einem einwertigen Kohlenwasserstoffrest besteht, der von 1 bis 20 Kohlenstoffatome enthält;
$R^2$ aus einer Gruppe ausgewählt wird, die aus einem zweiwertigen linearen oder verzweigten Kohlenwasserstoffrest besteht, der aus 1 bis 60 Kohlenstoffen besteht;
$R^4$ ein Kohlenwasserstoffrest ist, der 3 bis 200 Kohlenstoffatome enthält;
$R^5$ aus einer Gruppe ausgewählt wird, die aus Sauerstoff oder einem zweiwertigen linearen oder verzweigten Kohlenwasserstoffrest besteht, der aus 1 bis 60 Kohlenstoffen besteht;
$R^3$, $R^6$, $R^7$ und $R^8$ jeweils unabhängig aus der Gruppe einwertiger linearer oder verzweigter Kohlenwasserstoffradikale ausgewählt werden, die von 1 bis 200 Kohlenstoffatome aufweisen;
das tiefgestellte b 0 oder eine positive Zahl ist und einen Wert hat, der von 0 bis 3 reicht;
das tiefgestellte a eine positive Zahl kleiner als 3 ist;
die tiefgestellten b und c 0 oder positiv sind und einen Wert haben, der von 0 bis 3 reicht, mit der Einschränkung, dass $(a+b+c) \leq 3$;
die tiefgestellten d und e 0 oder positiv sind und einen Wert haben, der von 0 bis 3 reicht, mit der Einschränkung, dass $(d+e) = 3$.

**10.** Farbiger Haarschmuck nach Anspruch 9, wobei:
das Siloxan ein mit Epoxid endverkapptes Polysiloxan mit einer durchschnittlichen Struktur ist:

$CH_2(O)CHCH_2O(CH_2)_\lambda Si(CH_3)_2O[Si(CH_3)_2O]_kSi(CH_3)_2CH_2CH_2CH_2OCH_2C\ H(O)CH_2$, wobei das tiefgestellte k von 5 bis 250, von 5 bis 150, bevorzugt von 50 bis 100 reicht und das tiefgestellte $\lambda$ 2 oder 3 ist; und
das Aminosilan die Formel hat $H_2NR^2Si(OR^3)_2(OR^4)$, wobei
$R^2$ von 2 bis 5 Kohlenstoffatome hat;
$R^3$ von 3 bis 5 Kohlenstoffatome hat; und
$R^4$ von 3 bis 5 Kohlenstoffatome hat.

**11.** Farbiger Haarschmuck nach Anspruch 10, wobei das filmbildende Aminosilikonpolymer das Produkt der Reaktion von Siloxan (a1), Aminosilan (a2) und eines endverkappten Polyethers mit der durchschnittlichen Struktur $CH_2(O)CHCH_2O(CH_2CH_2O)_\mu CH_2CH(O)CH_2$ ist, wobei das tiefgestellte $\beta$ von 10 bis 20 reicht, oder ein endverkapptes Polyether mit der durchschnittlichen Struktur $CH_2(O)CHCH_2O(CH_2CH_2O)_\mu(CH_2CHCH_3O)_\nu CH_2CH(O)CH_2$, wobei das tiefgestellte $\mu$ von 1 bis 20 reicht und wobei das tiefgestellte $\nu$ von 1 bis 20 reicht.

**12.** Farbiger Haarschmuck nach einem der vorhergehenden Ansprüche, wobei das Haarfärbemittel ein oder mehrere Pigmente umfasst, wobei die ein oder mehreren Pigmente bevorzugt einen Partikeldurchmesser $D_{50}$ von 5 Mikron bis 60 Mikron aufweisen, wobei die ein oder mehreren Pigmente bevorzugter metallische Pigmente sind.

**13.** Farbiger Haarschmuck nach einem der vorhergehenden Ansprüche, wobei das filmbildende Aminosilikonpolymer der Haarfärbezusammensetzung Folgendes umfasst:
(c) ein Verdickungssystem, umfassend:

a. einen Abscheidungsverbesserer, wobei der Abscheidungsverbesserer ein hydrophiles und nicht-ionisches Polymer ist und wobei der Abscheidungsverbesserer ein gewichtsgemitteltes Molekulargewicht von 700.000 Dalton bis 3.000.000 Dalton aufweist;
b. ein Verdickungspolymer, wobei das Verdickungspolymer ein gewichtsgemitteltes Molekulargewicht von mindestens 10.000 Dalton aufweist und wobei das Verdickungspolymer ein kationisches Verdickungspolymer oder ein nicht-ionisches Verdickungspolymer ist.

**14.** Verfahren zum individuellen Anpassen eines farbigen Haarschmucks (100) nach einem der Ansprüche 1 bis 13, umfassend mindestens eines oder jedwede Kombination von zwei oder mehr von:

mechanisches Ändern der Oberfläche von mindestens einem der Vielzahl von lichtdurchlässigen Filamenten;
Schneiden von mindestens einem der Vielzahl von lichtdurchlässigen Filamenten;
Auftragen eines Haarfärbemittels oder einer Haarfärbezusammensetzung auf die Oberfläche von mindestens einem der Vielzahl von lichtdurchlässigen Filamenten; und
Anwenden einer Wärmebehandlung auf mindestens eines der Vielzahl von lichtdurchlässigen Filamenten,
wobei die individuelle Anpassung bevorzugt nach Anbringen des Haarschmucks am Kopf des Trägers durchgeführt wird.

**15.** Kit, umfassend:

(a) einen Haarschmuck (100), wobei der Haarschmuck eine Lichtquelle (110) und eine Vielzahl von lichtdurchlässigen Filamenten (10, 15, 130) umfasst; und wobei jedes lichtdurchlässige Filament ein proximales Ende (40, 132), ein distales Ende (50, 134) und eine Seitenwand hat, wobei das proximale Ende jedes lichtdurchlässigen Filaments so angeordnet ist, dass es Licht von der Lichtquelle empfängt; und
(b) eine Haarfärbezusammensetzung (80), wobei die Haarfärbezusammensetzung ein filmbildendes Aminosilikonpolymer (70) und ein Haarfärbemittel (75) umfasst;
(c) optional einen Applikator, um ein oder mehrere lichtdurchlässige Filamente der Vielzahl von lichtdurchlässigen Filamente mit der Haarfärbezusammensetzung zu beschichten.

## Revendications

**1.** Ornement capillaire coloré (100) comprenant :

une source de lumière (110) ; et
une pluralité de filaments transmettant la lumière (10, 15, 130) ;
où chaque filament transmettant la lumière a une extrémité proximale (40, 132), une extrémité distale (50, 134) et une paroi latérale, où l'extrémité proximale de chaque filament transmettant la lumière est conçue de manière à recevoir de la lumière provenant de la source de lumière ;
où un ou plusieurs filaments transmettant la lumière de la pluralité de filaments transmettant la lumière sont revêtus d'une composition de coloration capillaire (80) ;
où la composition de coloration capillaire comprend un polymère d'aminosilicone filmogène (70) et un agent de coloration capillaire (75).

**2.** Ornement capillaire coloré selon la revendication 1, dans lequel un revêtement réfléchissant est appliqué sur l'extrémité distale de chaque filament transmettant la lumière dépassant de la source de lumière.

**3.** Ornement capillaire coloré selon la revendication 1, dans lequel l'extrémité distale qui dépasse et s'éloigne de la source de lumière est coupée à un angle afin de maximiser la luminosité observée de la lumière émise par la source de lumière, où l'angle est compris entre 15 et 75 degrés.

**4.** Ornement capillaire coloré selon la revendication 1, dans lequel chaque filament transmettant la lumière est configuré pour émettre à travers la paroi latérale au moins une partie de la lumière reçue au niveau de l'extrémité proximale, le filament transmettant la lumière comprenant un premier segment de longueur unitaire, situé à une première distance de l'extrémité proximale, le premier segment étant configuré pour émettre à travers la paroi latérale une première proportion de la lumière arrivant au premier segment à partir de l'extrémité proximale ;
le filament transmettant la lumière comprenant en outre un second segment de longueur unitaire situé à une seconde distance de l'extrémité proximale, le second segment étant configuré pour émettre à travers la paroi latérale une seconde proportion de la lumière arrivant au second segment à partir de l'extrémité proximale ;
où la première distance est de préférence supérieure à la seconde distance ; et
où une propriété optique du filament transmettant la lumière varie sur une longueur du filament de sorte que la première proportion soit supérieure à la seconde proportion.

**5.** Ornement capillaire coloré selon l'une quelconque des revendications précédentes, dans lequel le filament transmettant la lumière comprend une âme et une gaine, de préférence dans lequel l'âme est fabriquée en PMMA.

**6.** Ornement capillaire coloré selon la revendication 5, dans lequel le filament transmettant la lumière comprend un revêtement constitué d'un polymère fluoré.

**7.** Ornement capillaire coloré selon l'une quelconque des revendications précédentes, dans lequel le filament transmettant la lumière comprend une couche incluant au moins un élément parmi : la kératine ; et la mélanine.

**8.** Ornement capillaire coloré selon l'une quelconque des revendications précédentes, dans lequel le polymère d'aminosilicone filmogène de la composition de coloration capillaire comprend :

(a) un polymère d'aminosilicone comprenant des chaînes latérales amino et où le polymère d'aminosilicone a un poids moléculaire moyen en poids de 10 000 à 60 000 daltons ; et
(b) une résine de silicone, de préférence une résine MQ.

**9.** Ornement capillaire coloré selon l'une quelconque des revendications 1 à 7, dans lequel le polymère d'aminosilicone filmogène est le produit de la réaction de :

a1) un siloxane comprenant au moins deux groupes oxirane ou oxétane ayant la formule :

$$M_f M^E{}_h M^{PE}{}_i M^H j D_k D^E{}_l D^{PE}{}_m D^H{}_n T_o T^E{}_p T^{PE}{}_q T^H{}_r Q_s$$

avec

$M = R^9R^{10}R^{11}SiO_{1/2}$;
$M^H = R^{12}R^{13}HSiO_{1/2}$;
$M^{PE} = R^{12}R^{13} (-CH_2CH(R^{14})(R^{15})_tO(R^{16})_u(C_2H_4O)_v(C_3H_6O)_w(C_4H_8O)_xR^{17})SiO_{1/2}$;
$M^E = R^{12}R^{13}(R^E)SiO_{1/2}$;
$D= R^{18}R^{19}SiO_{2/2}$ ; et
$D^H = R^{20}HSiO_{2/2}$;
$D^{PE} = R^{20} (-CH_2CH(R^{14})(R^{15})_tO(R^{16})_u(C_2H_4O)_v(C_3H_6O)_w(C_4H_8O)_xR^{17})SiO_{2/2}$;
$D^E = R^{20}R^ESiO_{2/2}$;
$T = R^{21}SiO_{3/2}$;
$T^H = HSiO_{3/2}$;
$T^{PE} = (-CH_2CH(R^{14})(R^{15})_tO(R^{16})_u(C_2H_4O)_v(C_3H_5O)_w(C_4H_8O)_xR^{17}) SiO_{3/2}$;
$T^E = R^ESiO_{3/2}$ ; et
$Q = SiO_{4/2}$ ;
Où
$R^9$, $R^{10}$, $R^{11}$, $R^{12}$, $R^{13}$, $R^{18}$, $R^{19}$, $R^{20}$, et $R^{21}$ sont choisis chacun indépendamment dans le groupe des radicaux hydrocarbonés monovalents ayant 1 à 60 atomes de carbone ;
$R^{14}$ est H ou un groupe alkyle de 1 à 6 atomes de carbone ;
$R^{15}$ est un radical alkyle divalent de 1 à 6 atomes de carbone ;
$R^{16}$ est choisi dans le groupe des radicaux divalents consistant en $-C_2H_4O-$, $-C_3H_6O-$, et $-C_4H_8O-$ ;
$R^{17}$ est choisi dans le groupe consistant en H, les radicaux hydrocarbonés monofonctionnels de 1 à 6 atomes de carbone et l'acétyle ;
$R^E$ est indépendamment un radical hydrocarboné monovalent contenant un ou plusieurs fractions oxirane ou oxétane ayant 1 à 60 atomes de carbone ;
l'indice f est égal à 0 ou positif sous réserve que, lorsque l'indice f est égal à 0, h doit être positif ;
l'indice h est égal à 0 ou positif, sous réserve que, lorsque h est égal à 0, l'indice f soit positif et que la somme des indices h, l et p soit positive ;
l'indice k est zéro ou positif et a une valeur comprise entre 0 et 1 000 ;
l'indice l est égal à 0 ou positif et a une valeur comprise entre 0 et 400, sous réerve que la somme des indices h, l et p soit positive ;
l'indice o est égal à 0 ou positif et a une valeur comprise entre 0 et 50 ;
l'indice p est égal à 0 ou positif et a une valeur comprise entre 0 et 30, sous réserve que la somme des indices h, l et p soit positive ;
l'indice s est égal à 0 ou positif et a une valeur comprise entre 0 et 20 ;
l'indice i est égal à 0 ou positif et a une valeur comprise entre 0 et 20 ;
l'indice m est égal à 0 ou positif et a une valeur comprise entre 0 et 200 ;
l'indice q est égal à 0 ou positif et a une valeur comprise entre 0 et 30 ;
l'indice j est égal à 0 ou positif et a une valeur comprise entre 0 et 2 ;
l'indice n est égal à 0 ou positif et a une valeur comprise entre 0 et 20 ;
l'indice r est égal à 0 ou positif et a une valeur comprise entre 0 et 30 ;
l'indice t est 0 ou 1 ;
l'indice u est 0 ou 1 ;
l'indice v est égal à 0 ou positif et a une valeur comprise entre 0 et 100, sous réserve de la limitation que $(v + w + x) > 0$ ;
l'indice w est égal à 0 ou positif et a un valeur comprise entre 0 et 100, sous réserve de la limitation que $(v + w + x) > 0$ ;

l'indice x est égal à 0 ou positif et a un valeur comprise entre 0 et 100, sous réserve de la limitation que (v + w + x) > 0 ; et

a2) un aminosilane ayant la formule :

$$N(H)(R^1)R^2Si(OR^3)_{3-a-b-c}(OR^4)_a(R^5Si(OR^6)_d(R^7)_e)_bR^8_c$$

avec

$R^1$ est choisi dans le groupe consistant en H ou un radical hydrocarboné monovalent contenant 1 à 20 atomes de carbone ;
$R^2$ est choisi dans un groupe consistant en un radical hydrocarboné linéaire ou ramifié divalent consistant en 1 à 60 atomes de carbone ;
$R^4$ est un radical hydrocarboné contenant de 3 à 200 atomes de carbone ;
$R^5$ est choisi dans un groupe consistant en l'oxygène ou un radical hydrocarboné linéaire ou ramifié divalent consistant en 1 à 60 atomes de carbone ;
$R^3$, $R^6$, $R^7$ et $R^8$ sont chacun indépendamment choisis dans le groupe des radicaux hydrocarbonés linéaires ou ramifiés monovalents ayant de 1 à 200 atomes de carbone ;
l'indice b est égal à 0 ou à un nombre positif et a une valeur comprise entre 0 et 3 ;
l'indice a est un nombre positif inférieur à 3 ;
les indices b et c sont 0 ou positifs et ont une valeur comprise entre 0 et 3 sous réserve de la limitation que $(a + b + c) \leq 3$ ;
les indices d et e sont 0 ou positifs et ont une valeur comprise entre 0 et 3 sous réserve de la limitation que $(d + e) = 3$.

**10.** Ornement capillaire coloré selon la revendication 9, dans lequel :
le siloxane est un polysiloxane enrobé d'époxy de structure moyenne :

$CH_2(O)CHCH_2O(CH_2)_\lambda Si(CH_3)_2O[Si(CH_3)_2O]_kSi(CH_3)_2CH_2CH_2CH_2OCH_2C\ H(O)CH_2$ avec l'indice k compris entre 5 et 250, entre 5 et 150, de préférence entre 50 et 100, et l'indice $\lambda$ égal à 2 ou 3 ; et
l'aminosilane a la formule $H_2NR^2Si(OR^3)_2(OR^4)$ avec
$R^2$ a de 2 à 5 atomes de carbone ;
$R^3$ a de 3 à 5 atomes de carbone ; et
$R^4$ a de 3 à 5 atomes de carbone.

**11.** Ornoment capillaire coloré selon la revendication 10, dans lequel le polymère filmogène d'aminosilicone est le produit de la réaction du siloxane (a1), de l'aminosilane (a2) et d'un polyéther enrobé de structure moyenne $CH_2(O)CHCH_2O(CH_2CH_2O)_\mu CH_2CH(O)CH_2$ avec l'indice $\beta$ compris entre 10 et 20, ou un polyéther enrobé de structure moyenne $CH_2(O)CHCH_2O(CH_2CH_2O)_\mu(CH_2CHCH_3O)_\nu CH_2CH(O)CH_2$ avec l'indice $\mu$ compris entre 1 et 20 et avec l'indice $\nu$ compris entre 1 et 20.

**12.** Ornement capillaire coloré selon l'une quelconque des revendications précédentes, dans lequel l'agent de coloration capillaire comprend un ou plusieurs pigments, de préférence où le ou les pigments ont un diamètre de particule $D_{50}$ de 5 microns à 60 microns, plus préférablement où le ou les pigments sont des pigments métalliques.

**13.** Ornement capillaire coloré selon l'une quelconque des revendications précédentes, dans lequel le polymère d'aminosilicone filmogène de la composition de coloration capillaire comprend :
(c) un système épaississant comprenant :

a. un activateur de dépôt, où l'activateur de dépôt est un polymère hydrophile et non ionique, et où l'activateur de dépôt a un poids moléculaire moyen en poids de 700 000 daltons à 3 000 000 daltons ;
b. un polymère épaississant, où le polymère épaississant a un poids moléculaire moyen en poids d'au moins 10 000 daltons et où le polymère épaississant est un polymère épaississant cationique ou un polymère épaississant non ionique.

**14.** Procédé de personnalisation d'un ornement capillaire coloré (100) selon l'une quelconque des revendications 1 à 13, comprenant au moins une ou n'importe laquelle des combinaisons de deux ou plus des éléments suivants :

la modification mécanique de la surface d'au moins un de la pluralité de filaments transmettant la lumière ;
la découpe d'au moins l'un de la pluralité de filaments transmettant la lumière ;
l'application d'un agent de coloration capillaire ou une composition de coloration capillaire sur la surface d'au moins un de la pluralité de filaments transmettant la lumière ; et
l'application d'un traitement thermique à au moins un de la pluralité de filaments transmettant la lumière,
où ladite personnalisation est de préférence effectuée après la fixation de l'ornement capillaire sur la tête de l'utilisateur.

**15.** Un kit comprenant :

(a) un ornement capillaire (100), dans lequel l'ornement capillaire comprend une source de lumière (110) et une pluralité de filaments transmettant la lumière (10, 15, 130) ; où chaque filament transmettant la lumière a une extrémité proximale (40, 132), une extrémité distale (50, 134) et une paroi latérale, où l'extrémité proximale de chaque filament transmettant la lumière est conçue de manière à recevoir de la lumière provenant de la source de lumière ; et
(b) une composition de coloration capillaire (80), où la composition de coloration capillaire comprend un polymère d'aminosilicone filmogène (70) et un agent de coloration capillaire (75) ;
(c) éventuellement un applicateur pour revêtir la composition de coloration capillaire sur un ou plusieurs filaments transmettant la lumière de la pluralité de filaments transmettant la lumière.

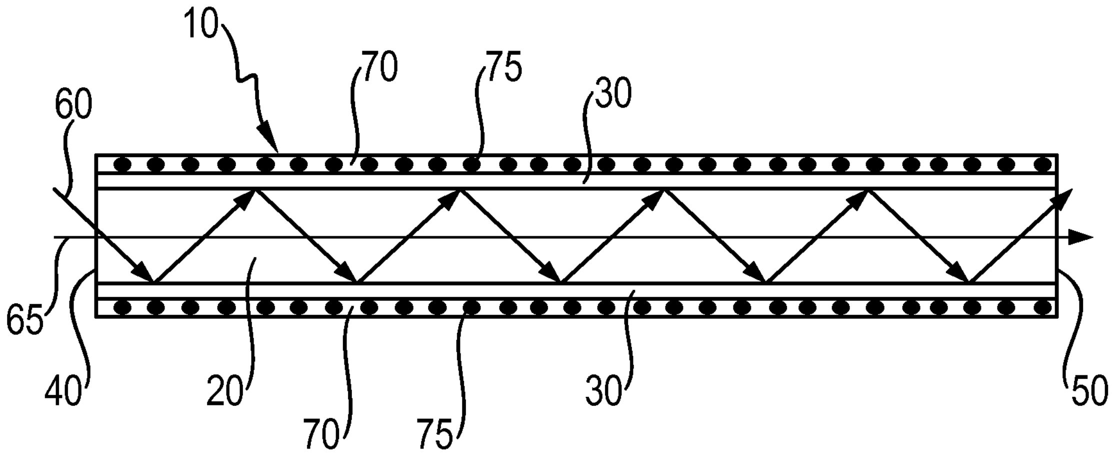


Fig. 1

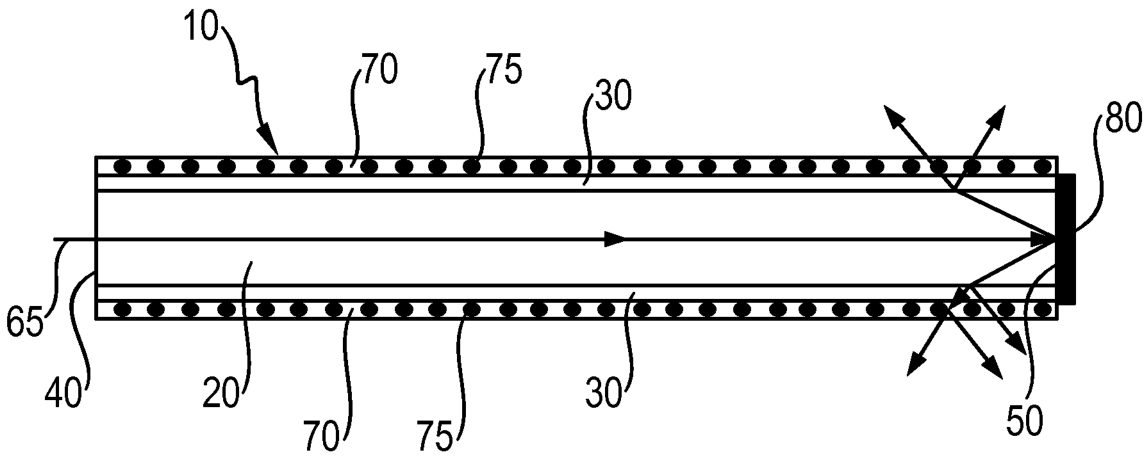


Fig. 2

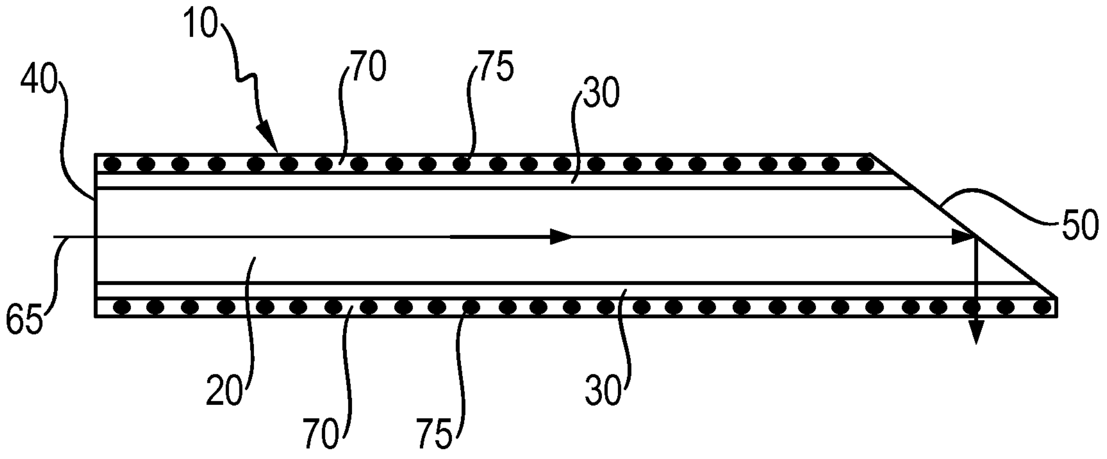
10
40
70
75
30
50
65
20
70
75
30

Fig. 3

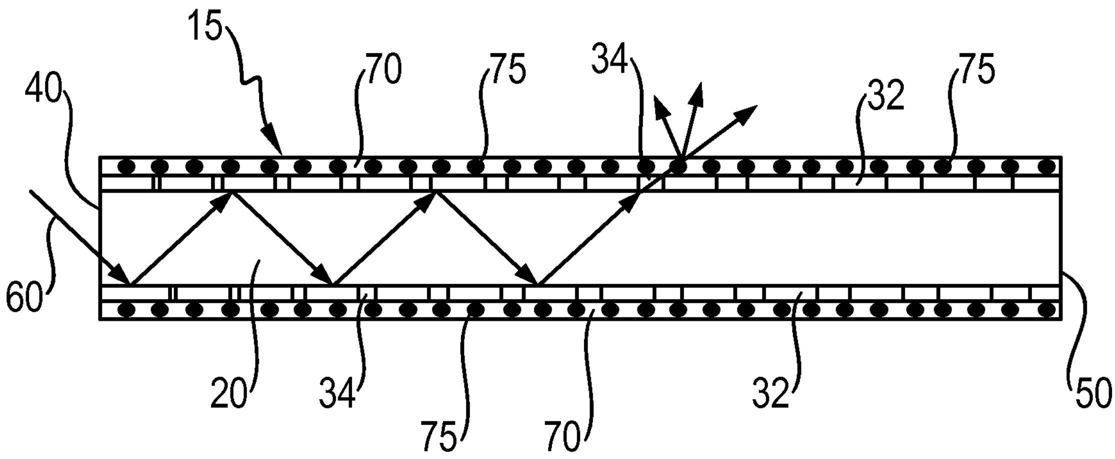
15
40
70
75
34
32
75
60
20
34
75
70
32
50

Fig. 4

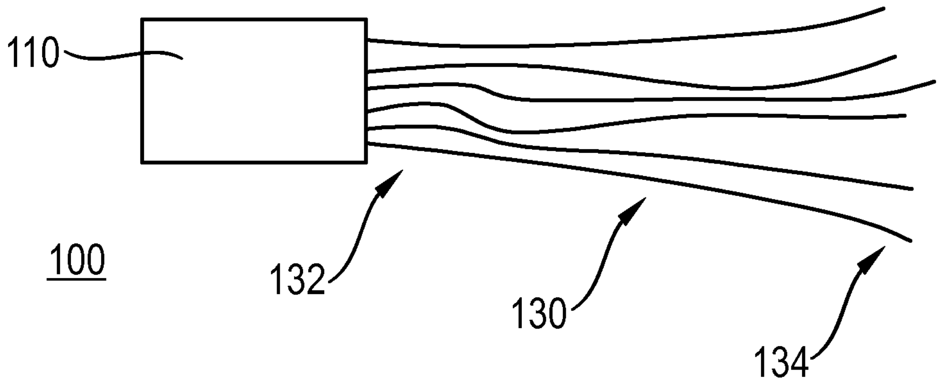


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3675005 A
- US 2003156429 A1
- US 6451747 B1, Decoster
- US 5567428 A, Hughes
- US 20040010863 A1, Gawtrey
- US 20060041026 A1, Mahr
- EP 2214633 B1
- EP 1084696 A1


### Non-patent literature cited in the description


- **BARBER et al.** *Pharmaceutical Development and Technology,* 1998, vol. 3 (2), 153-161
- International Cosmetics Ingredient Dictionary and Handbook. The Cosmetics, Toiletry, and Fragrance Association